# EUROPEAN PATENT APPLICATION

(11) **EP 1 283 039 A1**
(43) Date of publication of application: **12.02.2003**
(21) Application number: 01932128.0
(22) Date of filing: 18.05.2001
(51) Int. Cl.: A61K 31/135, A61K 31/14, A61K 31/165, A61K 31/40, A61K 31/4453, A61K 31/4164, A61P 43/00, A61P 25/00, A61P 25/16, A61P 25/28

(54) **-SECRETASE INHIBITORS**

(30) Priority: 19.05.2000 JP 2000152758
(71) Applicant: Takeda Chemical Industries, Ltd., Osaka-shi, Osaka 541-0045 (JP)
(72) Inventor: MIYAMOTO, Masaomi, Takarazuka-shi, Hyogo 665-0841 (JP); MATSUI, Junji, Higashiyodogawa-ku Osaka-shi, Osaka (JP); FUKUMOTO, Hiroaki, Kawabe-gun, Hyogo 666-0257 (JP); TARUI, Naoki, Nara-shi, Nara 631-0061 (JP)
(74) Representative: Wright, Robert Gordon McRae
(86) International application number: JP0104144
(87) International publication number: WO01087293

(57) **Abstract**

An excellent β secretase inhibitor is provided, which comprises a compound of the general formula: 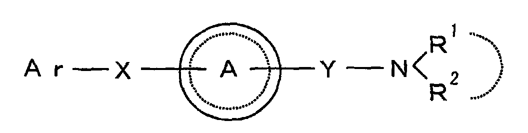 wherein Ar is an aromatic group; X is a divalent group selected from -O-, -S-, -CO-, -SO-, -SO₂-, -NR⁸-, -CONR⁸-, -SO₂NR⁸-, and -COO- (wherein R⁸ is hydrogen, etc.), a divalent C₁₋₆ aliphatic hydrocarbon group which may contain one or two of these divalent groups, or a bond; Y is a divalent group selected from -O-, -S-, -CO-, -SO-, -SO₂-, -NR⁸-, -CONR⁸-, -SO₂NR⁸-, and -COO-, or a divalent C₁₋₆ aliphatic hydrocarbon group which may contain one or two of these divalent groups; R¹ and R² are hydrogen, a hydrocarbon group, etc., respectively; and A is a ring which may be further substituted, or a salt thereof.

## Description

### Technical Field

The present invention relates to β secretase inhibitors having excellent properties.

### Background Art

Alzheimer's disease is a nerve degenerative disease characterized by formation of senile plaques and neurofibrillary tnagles together with degeneration and disappearance of nerve cells. The senile plaques that is most characteristic of Alzheimer's disease are deposits consisting primarily of β amyloid protein (also referred to hereinafter as Aβ) in the brain (Biochem. Biophys. Res. Commun., 122: 1131 (1984)). Aβ consisting of 40 or 42 amino acids (hereinafter abbreviated as Aβ1-40 and Aβ1-42, respectively) is known to be toxic to nerve cells (Trend in Neuroscience (TINS), 16: 409 (1993); Science, 274: 99 (1996); Nature, 395: 755 (1998); Neurobiology of Aging, 20: 201 (1999)).

Accordingly, drugs inhibiting production and secretion of Aβ are effective in preventing and/or treating diseases attributable to Aβ (e.g., Alzheimer's disease, Down's syndrome etc.). By development of enzyme immunoassay (EIA) of Aβ, not only screening of compounds inhibiting secretion of Aβ from cultured nerve cells but also quantification of Aβ in various biological tissues, blood and cerebrospinal fluid is made feasible in recent years (e.g., Science, 264: 1336 (1994); Biochemistry, 34: 10272 (1995); Science, 274: 99 (1996)).

In production of Aβ, its precursor protein APP (amyloid precursor protein) is cleaved with β secretase and γ secretase. Recently, isolation of β secretase cDNA was reported and β secretase was revealed in some laboratories (e.g., Science, 286: 735 (1999); Nature, 402: 533 (1999); Nature, 402: 537 (1999)). Among patients with familial Alzheimer's disease, a mutation in the APP gene has been recognized, and in cells where the gene undergoes this mutation, an increase in the amount of Aβ produced and secreted is noted (e.g., Nature, 360: 672 (1992); Science, 259: 514 (1993); Science, 264: 1336 (1994)). It is therefore expected that for patients with increased levels of Aβ protein in the brain, such as patients hereditarily liable to have diseases attributable to Aβ, such as familial Alzheimer's disease (e.g., Alzheimer's disease, Down's syndrome etc.) or patients with increased Aβ protein in the brain by injuries etc., drugs inhibiting β secretase, inhibit production and secretion of Aβ are useful for prophylactic and therapeutic agents for the diseases.

Further, when β secretase is inhibited, a metabolic pathway for producing Aβ from APP is inhibited and suppressed, while secretion of secretory APP (abbreviated as sAPPα) produced by α secretase is promoted. sAPPα is reported to have a neurotrophic factor-like action (Neuron, 10: 243-254 (1993); Trend in Neuroscience (TINS), 16: 409 (1993)). As the neurotrophic factor-like actions, (1) an action for survival and maintenance of nerve cells, (2) a promoting action on formation of synapses, (3) a protective action on nerve cells against death, and (4) a long and enhancing action in the hippocampus. It is therefore considered that the drugs inhibiting β secretase are also useful by promoting secretion of sAPPα for preventing and treating (1) nerve degenerative diseases, (2) nerve disorders at the time of cerebrovascular disorders, at the time of head or spinal cord injuries, at the time of the sequelae of encephalitis, or at the time of cerebral palsy, (3) memory impairment, or (4) psychiatric disorders.

On one hand, JP 11-80098 A describes an inhibitor of amyloid β protein production and secretion, which comprises a compound represented by the formula: 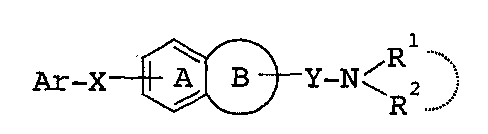 wherein Ar' is an aromatic group which may be substituted; X represents (i) a bond, (ii) -S-, -SO- or -SO₂-, (iii) C₁₋₆ alkylene, C₂₋₆ alkenylene or C₂₋₆ alkynylene, each of which may have 1 to 3 substituents selected from oxo and C₁₋₆ alkyl, (iv) -CO-O-, or (v) a group represented by the formula -(CH₂)p-X¹-, -(CH₂)p-X¹-(CH₂)q-, -(CH₂)r-CO-X¹-, -SO₂-NR⁸- or -(CH₂)r-SO₂-NR⁸- (wherein X¹ represents oxygen atom or NR⁸, R⁸ represents hydrogen atom or a hydrocarbon group or acyl which may be substituted, p is an integer of 0 to 5, q is an integer of 1 to 5, p + q is an integer of 1 to 5, and r is an integer of 1 to 4); Y represents a divalent C₁₋₆ aliphatic hydrocarbon group which may be bound via oxygen atom or sulfur atom and which may be substituted; R¹ and R² represent a hydrogen atom or a lower alkyl group which may be substituted, respectively, or R¹ and R² may, together with the adjacent nitrogen atom, form a nitrogen-containing heterocyclic ring which may be substituted; ring A represents benzene ring which may be substituted in addition to the group represented by the formula -X-Ar wherein each symbol is as defined above; ring B represents a 4- to 8-membered ring which may further be substituted in addition to the group represented by the formula -Y-NR¹R² wherein each symbol is as defined above, or a salt thereof.

EP-A-754455 describes that a compound represented by the formula: 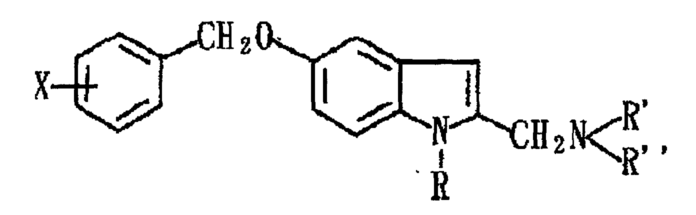 wherein X represents hydrogen atom, halogen, alkoxy, alkyl, alkylthio, aryl or aryloxy; R represents hydrogen atom, CH₃ or other aliphatic, non-cyclic or aryl radicals; R' represents hydrogen atom, etc.; and R'' represents hydrogen atom, etc., or a salt thereof, has an inhibitory activity on monoamine oxidase (MAO) and useful as a nerve protecting agent in central nerve degenerative diseases (Parkinson's disease, Alzheimer's disease etc.).

JP 2-96552 A (USP 5,137,901) describes that a compound represented by the formula: 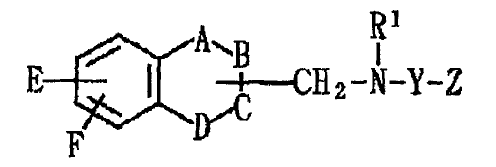 wherein Y represents a linear or branched, substituted or unsubstituted alkylene chain having up to 6 carbon atoms; Z is a group of the formula -NR₂R₃, -OR₄ or the like; R₂ and R₃ are the same or different and represent hydrogen, alkyl, alkenyl or cycloalkyl, or aryl which may be substituted with halogen, etc.; R₄ represents hydrogen, alkyl or alkenyl, R₁ represents hydrogen, alkyl, aralkyl, heteroaryl alkyl or the group -(Y₁-Z₁) (Y₁ and Z₁ are the same or different and are as defined above with respect to Y and Z, respectively); A and D represent the formula -CH₂, O, S or NR₁₃, respectively, or the -CH or N moiety of a C=C or C=NH double bond, provided that A only or D only is oxygen, sulfur or N-R₁₃, R₁₃ is hydrogen, alkyl, alkoxy, acyl, alkoxycarbonyl or alkylsulfonyl; B is the formula -CH₂ or ≡CH, or the CH or N moiety of a C=C or C=N double bond; C is a group of the formula ≡CH or the C moiety of a C=C or C=N double bond; E and F are the same or different and represent hydrogen, alkyl, alkoxy, halogen, nitro, cyano, trifluoromethyl, trifluoromethoxy or a group of the formula -CONR₂R₃ (R₂ and R₃ are as described above), respectively, or E and F are combined to form a substituted or unsubstituted carbon ring having 6 carbon atoms, acts as an agonist, a partial agonist and an antagonist of serotonin receptors, and is suitable for treatment of central nerve diseases, etc.

JP 63-77842 A describes that a compound represented by the formula: 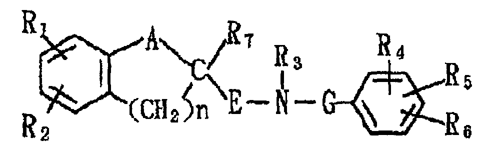 wherein n is 1 or 2; A is carbonyl and R₇ is hydrogen atom, or A is the formula: -CHR₈- (R₈ represents hydrogen atom, alkanoyloxy or alkoxycarbonyl) and R₇ is hydrogen atom, or R₇ and R₈ are combined to form another bond; E represents a C₃₋₄ linear alkylene which may be substituted with alkyl; G represents a C₂₋₅ linear alkylene which may be substituted with alkyl; R₁ represents hydrogen atom, trifluoromethyl, nitro, amino, alkylamino, dialkylamino, alkyl group, hydroxy, alkoxy or phenylalkoxy, R₂ represents hydrogen, halogen atom, hydroxy, alkoxy, phenylalkoxy or alkyl, or R₁ and R₂ are combined to form a C₁ or C₂ alkylenedioxy; R₃ represents hydrogen atom, C₃₋₅ alkenyl or alkyl; R₄ represents hydrogen atom, halogen atom, alkyl, etc.; R₅ represents hydrogen atom, halogen atom, alkyl, etc.; R₆ represents hydrogen atom, halogen atom, alkyl, etc., has an activity of decreasing heart rate and an activity of reducing O₂ requirement in the heart, and is suitable for treating sinus tachycardia and preventing and treating ischemic heart diseases.

WO 92/15558 describes that a compound represented by the formula: 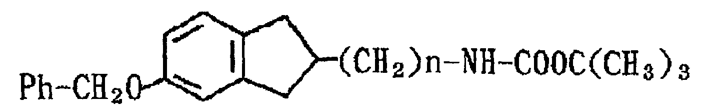 wherein n is an integer of 1 to 4, serves as an intermediate for a compound having an antagonistic action on thromboxane A2.

WO 95/32967 describes that an amide derivative represented by the formula: 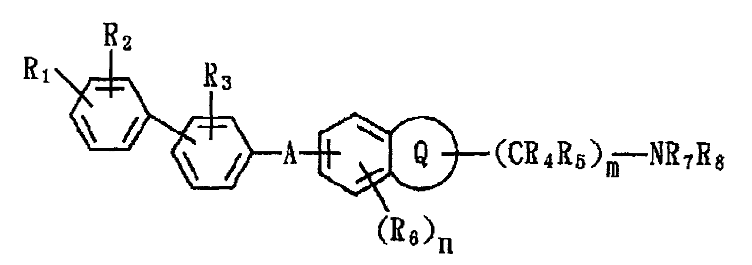 wherein A represents CONR, R represents hydrogen atom or C₁₋₆ alkyl; Q represents a 5- to 7-membered ring containing 1 to 3 heteroatoms selected from oxygen atom, nitrogen atom and sulfur atom; R₁ represents hydrogen atom, halogen atom, etc.; R₂ and R₃ represent hydrogen atom, halogen, etc., respectively; R₄ and R₅ represent hydrogen atom or C₁₋₆ alkyl, respectively, R₆ represents halogen, etc., R₇ and R₈ represent hydrogen atom, C₁₋₆ alkyl, or aralkyl, respectively, or are combined with the adjacent nitrogen atom, form a 5- to 7-membered heterocyclic ring which may be substituted and contains 1 to 2 heteroatoms selected from oxygen atom, nitrogen atom and sulfur atom; m is 0 to 4; and n is 0, 1 or 2, has an antagonistic action on 5HT1D, and is useful as treatment of central nerve diseases.

JP 2-91052 A describes a choline esterase inhibitor comprising an substituted amine represented by the formula: 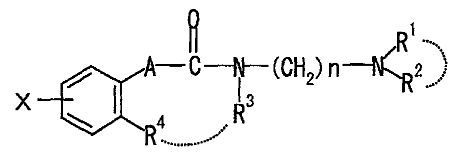 wherein R¹ and R² independently represent hydrogen atom or a hydrocarbon residue which may be substituted, or together with the adjacent nitrogen atom, form a condensed heterocyclic group; R³ and R⁴ are such that R³ represents hydrogen atom, or a hydrocarbon residue or an acyl group, each of which may be substituted, and R⁴ represents hydrogen atom, or R³ and R⁴ are combined to form -(CH₂)m-CO-, -CO-(CH₂)m- or -(CH₂)m+1- (m is an integer of 0, 1 or 2); A represents -(CH₂)l- (l is an integer of 0, 1 or 2) or -CH=CH-; X represents one or more substituents; and n is an integer of 4 to 7, or a salt thereof.

JP 2-73069 A discloses thiazole derivatives having an inhibitory activity of monoamine oxidase.

JP 5-239005 A discloses N-(2-aminoethyl)benzamides useful for treatment of senile dementia.

JP 9-507069 A discloses a compound having an inhibitory activity of phospholipase A₂ and useful for treatment of senile dementia.

WO 91/19697 discloses pyridine derivatives having an antagonistic activity of angiotensin II.

JP 7-508038 A (WO 93/23040) discloses 17-ether and thioether of a 4-azasteriod which have an inhibitory activity of 5α-reductase.

USP 6,048,877 describes a method of treating cardiac arrhythmia by administering a tetralone derivative.

WO 98/06691 describes that dendritic compounds including amine compounds having a naphthalene ring are useful for treatment of a wide range of diseases such as cancers, Alzheimer's disease, thrombosis, inflammatory diseases and microbial resistance.

JP 6-503948 A (WO 92/03542) describes a proteolytic factor having an ability to cleave a β protein precursor at outside a β protein domain and close to the N-terminus of β protein.

JP 8-502587 A (WO 94/10569) describes a method of identifying inhibitors of β amyloid peptide (βAP) production.

JP 7-165606 A describes a method of identifying inhibitors of βAP production.

JP 10-509797 A (WO 96/15452) describes a method of detecting a soluble amyloid β peptide in a liquid sample.

JP 11-507538 A (WO 96/40885) describes a composition comprising an isolated and purified enzyme cleaving a β amyloid precursor protein specifically at a cleavage position of a β amyloid peptide.

JP 9-178743 A describes a method of quantifying soluble APP characterized by using an antibody against an amyloid β protein or a soluble amyloid precursor protein (APP).

There is demand for development of compounds having an excellent inhibitory action on β secretase and sufficiently satisfactory as a pharmaceutical preparation.

### Disclosure of the Invention

The present inventors made extensive study on compounds having an inhibitory activity of β secretase, and as a result, they unexpectedly found that a compound represented by the formula: 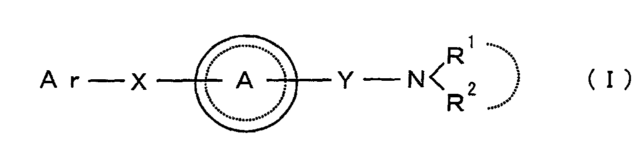 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or a hydrocarbon group which may be substituted, or R¹ and R² may, together with their adjacent nitrogen atom, form a nitrogen-containing heterocyclic ring which may be substituted; and ring A represents a ring which may further be substituted [hereinafter referred to as Compound (I)], or a salt thereof, has an excellent inhibitory activity of β secretase. As a result of further extensive study on the basis of these findings, the present inventors completed the present invention.

That is, the present invention relates to:
(1) A β secretase inhibitor comprising Compound (I), or a salt thereof;
(2) The β secretase inhibitor according to the above (1), which comprises a compound represented by the formula: 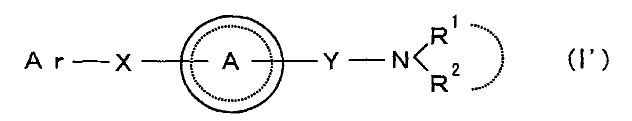 wherein Ar represents an aromatic group which may be substituted; X represents a divalent group selected from -O-, -S-, -CO-, -SO-, -SO₂-, -NR⁸-, -CONR⁸-, -SO₂NR⁸- and COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or a hydrocarbon group which may be substituted, respectively, or R¹ and R² may, together with the adjacent nitrogen atom, form a nitrogen-containing heterocyclic ring which may be substituted; and ring A represents an aromatic ring which may further be substituted (hereinafter sometimes referred to as Compound (I')), or a salt thereof;
(3) The inhibitor according to the above (1) which comprises a compound represented by the formula: 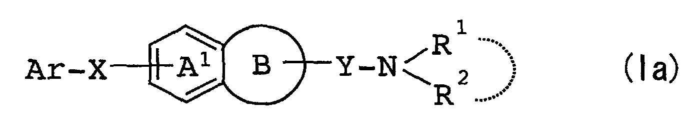 wherein Ar represents an aromatic group which may be substituted; X represents a divalent group selected from -O-, -S-, -CO-, -SO-, -SO₂-, -NR⁸-, -CONR⁸-, -SO₂NR⁸- and COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or C₁₋₆ alkyl which may be substituted, respectively, or R¹ and R² may, together with the adjacent nitrogen atom, form a nitrogen-containing heterocyclic ring which may be substituted; ring A¹ represents benzene ring which may further be substituted, and ring B represents a 4- to 8-membered ring which may further be substituted (hereinafter sometimes referred to as Compound (Ia)), or a salt thereof;
(4) The inhibitor according to the above (1) which comprises a compound represented by the formula: 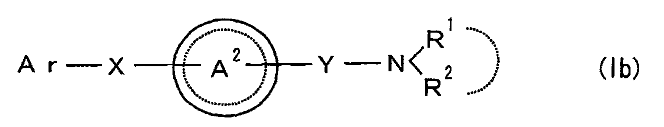 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or C₁₋₆ alkyl which may be substituted, respectively, or R¹ and R² may, together with the adjacent nitrogen atom, form a nitrogen-containing heterocyclic ring which may be substituted; and ring A² represents a monocyclic aromatic ring which may further be substituted (hereinafter sometimes referred to as Compound (Ib)), or a salt thereof;
(5) The inhibitor according to the above (1), wherein the aromatic group represented by Ar is a monocyclic aromatic group, a ring-assembled aromatic group or a condensed aromatic group;
(6) The inhibitor according to the above (1), wherein Ar is a ring-assembled aromatic group which may be substituted;
(7) The inhibitor according to the above (6), wherein the ring-assembled aromatic group is biphenylyl;
(8) The inhibitor according to the above (1), wherein X is -(CH₂)pO- (p is an integer of 1 to 3), -CONH-, -SO₂NH- or C₁₋₃ alkylene;
(9) The inhibitor according to the above (1), wherein Y is C₁₋₃ alkylene, -(CH₂)qCONR⁹(CH₂)r- (each of q and r is 0 to 3 and the sum of q and r is an integer of 3 or less, and R⁹ represents hydrogen atom, optionally halogenated C₁₋₆ alkyl or optionally halogenated C₁₋₆ alkyl-carbonyl) or -(CH₂)qCOO(CH₂)r- wherein the symbols are as defined above;
(10) The inhibitor according to the above (1), wherein the ring represented by ring A is a monocyclic aromatic ring or a condensed aromatic ring;
(11) The inhibitor according to the above (1), wherein the ring A is benzene ring, a 6-membered nitrogen-containing aromatic heterocyclic ring or tetralin ring, each of which may be substituted with a halogen atom and/or C₁₋₆ alkoxy;
(12) The inhibitor according to the above (1), wherein the ring A is a benzene ring or tetralin ring, each of which is di-substituted with the group represented by Ar-X- and the group represented by: 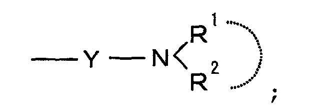
(13) The inhibitor according to the above (1), wherein Ar represents biphenylyl; X represents -(CH₂)pO- (p is an integer of 1 to 3), -CONH-, -SO₂NH- or C₁₋₃ alkylene; Y represents C₁₋₃ alkylene, -(CH₂)qCONH(CH₂)r- (each of q and r is 0 to 3 and the sum of q and r is an integer of 3 or less); R¹ and R² represent a hydrogen atom or C₁₋₆ alkyl, respectively, or R¹ and R² may, together with the adjacent nitrogen atom, form a 5- to 6-membered nitrogen-containing heterocyclic ring; and ring A represents benzene ring, a 6-membered nitrogen-containing aromatic heterocyclic ring or tetralin ring, each of which may be substituted with a halogen atom and/or C₁₋₆ alkoxy;
(14) The inhibitor according to the above (13), wherein the ring A is benzene ring or tetralin ring, each of which is di-substituted with the group represented by Ar-X- and the group represented by: 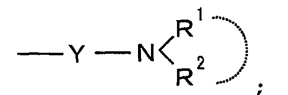
(15) The inhibitor according to the above (1) which is an agent for preventing and/or treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment, or (iv) psychiatric disorders, in which β secretase is involved;
(16) The inhibitor according to the above (1) which is an agent by promotion of secretion of sAPPα or due to both promotion of secretion of sAPPα and inhibition of production and secretion of β amyloid protein for preventing and/or treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment, or (iv) psychiatric disorders;
(17) The inhibitor according to the above (15) or (16), wherein the nerve degenerative disease is Alzheimer's disease or Parkinson's disease;
(18) The inhibitor according to the above (1) which is a stimulator of secretion of sAPPα;
(19) The inhibitor according to the above (1) which is a neurotrophic factor-like agent;
(20) The inhibitor according to the above (1) which is an agent for preventing and/or treating psychiatric disorders or nerve disorders at the time of head or spinal cord injuries, at the time of the sequelae of encephalitis, or at the time of cerebral palsy;
(21) Use of the β secretase inhibitor according to the above (1) for stimulation of an sAPPα secretion, a neurotrophic factor-like agent, or an agent for preventing and/or treating nerve disorders at the time of head or spinal cord injuries, at the time of the sequelae of encephalitis, or at the time of cerebral palsy, or memory impairment or psychiatric disorders; and
(22) A method for treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment or (iv) psychiatric disorders in which β secretase is involved, which comprises administrating an effective amount of the β secretase inhibitor according to the above (1) to a mammal.

The "optionally halogenated C₁₋₆ alkyl" used herein includes, for example, C₁₋₆ alkyl (e.g., methyl, ethyl, propyl, isopropyl, butyl, isobutyl, sec-butyl, tert-butyl, pentyl, hexyl, etc.) which may have 1 to 5, preferably 1 to 3 halogen atoms (e.g., fluorine, chlorine, bromine, iodine, etc.). Examples thereof include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

The "optionally halogenated C₃₋₆ cycloalkyl" used herein includes, for example, C₃₋₆ cycloalkyl (e.g., cyclopropyl, cyclobutyl, cyclopentyl, cyclohexyl, etc.) which may have 1 to 5, preferably 1 to 3 halogen atoms (e.g., fluorine, chlorine, bromine, iodine, etc.). Examples thereof include cyclopropyl, cyclobutyl, cyclopentyl, cyclohexyl, 4,4-dichlorocyclohexyl, 2,2,3,3-tetrafluorocyclopentyl, 4-chlorocyclohexyl, etc.

The "optionally halogenated C₁₋₆ alkoxy" used herein includes, for example, C₁₋₆ alkoxy (e.g., methoxy, ethoxy, propoxy, butoxy, pentyloxy, etc.) which may have 1 to 5, preferably 1 to 3 halogen atoms (e.g., fluorine, chlorine, bromine, iodine, etc.). Examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.

The "optionally halogenated C₁₋₆ alkylthio" used herein includes, for example, C₁₋₆ alkylthio (e.g., methylthio, ethylthio, propylthio, isopropylthio, butylthio, sec-butylthio, tert-butylthio, etc.) which may have 1 to 5, preferably 1 to 3 halogen atoms (e.g., fluorine, chlorine, bromine, iodine, etc.). Examples thereof include methylthio, difluoromethylthio, trifluoromethylthio, ethylthio, propylthio, isopropylthio, butylthio, 4,4,4-trifluorobutylthio, pentylthio, hexylthio, etc.

The "optionally halogenated C₁₋₆ alkyl-carbonyl" used herein includes, for example, C₁₋₆ alkyl-carbonyl (e.g., acetyl, propanoyl, butanoyl, pentanoyl, hexanoyl, etc.) which may have 1 to 5, preferably 1 to 3 halogen atoms (e.g., fluorine, chlorine, bromine, iodine, etc.). Examples thereof include acetyl, monochloroacetyl, trifluoroacetyl, trichloroacetyl, propanoyl, butanoyl, pentanoyl, hexanoyl, etc.

The "optionally halogenated C₁₋₆ alkylsulfonyl" used herein includes, for example, C₁₋₆ alkylsulfonyl (e.g., methylsulfonyl, ethylsulfonyl, propylsulfonyl, isopropylsulfonyl, butylsulfonyl, sec-butylsulfonyl, tert-butylsulfonyl, etc.) which may have 1 to 5, preferably 1 to 3 halogen atoms (e.g., fluorine, chlorine, bromine, iodine, etc.). Examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, hexylsulfonyl, etc.

The "optionally halogenated C₁₋₆ alkyl-carboxamide" used herein includes, for example, C₁₋₆ alkyl-carboxamide (e.g., acetamide, etc.) which may have 1 to 5, preferably 1 to 3 halogen atoms (e.g., fluorine, chlorine, bromine, iodine, etc.). Examples thereof include acetamide, trifluoroacetamide, propanamide, butanamide, etc.

In the above formulas, the aromatic group represented by Ar includes, for example, a monocyclic aromatic group, a ring-assembled aromatic group, a condensed aromatic group, etc.

The "monocyclic aromatic group" includes, for example, a monovalent group derived by removing an arbitrary hydrogen atom from a benzene ring or a 5- or 6-membered aromatic heterocyclic group.

The "5- or 6-membered aromatic heterocyclic ring" includes, for example, a 5- or 6-membered aromatic heterocyclic ring containing one or more (for example 1 to 3, preferably 1 to 2) heteroatoms selected from nitrogen atom, sulfur atom and oxygen atom in addition to the carbon atoms, or the like. Examples thereof include thiophene, furan, pyrrole, imidazole, pyrazol, thiazole, oxazole, pyridine, pyrazine, pyrimidine and pyridazine rings, etc.

Examples of the monocyclic aromatic group include phenyl, 2- or 3-thienyl, 2- or 3-furyl, 1-, 2- or 3-pyrrolyl, 2- or 4-imidazolyl, 3- or 4-pyrazolyl, 2-, 4- or 5-thiazolyl, 2-, 4- or 5-oxazolyl, 2-, 3- or 4-pyridyl, 2-pyrazinyl, 2-, 4- or 5-pyrimidinyl, 3- or 4-pyridazinyl, etc., among which phenyl or the like is preferable.

The "ring-assembled aromatic group" used is, for example, a group derived by removing an arbitrary hydrogen atom from an aromatic ring cluster wherein two or more (preferably two or three) aromatic rings are directly bound via a single bond and the number of direct bonds to the rings is smaller by one than the number of rings in the cyclic system. The "aromatic ring" includes an aromatic hydrocarbon, an aromatic heterocyclic ring, etc.

The "aromatic hydrocarbon" includes, for example, a C₆₋₁₄ monocyclic or condensed polycyclic (for example, di- or tricyclic) aromatic hydrocarbon (e.g., benzene, naphthalene, indene, anthracene, etc.), and the like.

The "aromatic heterocyclic ring" includes, for example, a 5- to 14-membered, preferably 5- to 10-membered aromatic heterocyclic ring containing one or more (for example 1 to 4, preferably 1 to 2) heteroatoms selected from nitrogen atom, sulfur atom and oxygen atom in addition to the carbon atoms, and the like. Examples thereof include aromatic heterocyclic rings such as thiophene, benzothiophene, benzofuran, benzimidazole, benzoxazole, benzothiazole, benzisothiazole, naphtho[2,3-b]thiophene, furan, phenoxathiine, pyrrole, imidazole, pyrazol, oxazole, isoxazole, 1,2,4-oxadiazole, 1,3,4-oxadiazole, 1,2,4-thiadiazole, 1,3,4-thiadiazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, furazane, phenoxazine, phthalimide, 2-, 3- or 4-pyridone, 2-, 3- or 4-quinolone, as well as a ring formed by condensing these rings (preferably monocycle) with one or more (preferably one or two) aromatic rings (e.g., benzene ring, etc.), and the like.

The aromatic ring cluster consisting of these aromatic rings linked directly via a single bond is, for example an aromatic ring cluster formed from 2 or 3 (preferably 2) aromatic rings selected from a benzene ring, a naphthalene ring and a 5- to 10-membered (preferably 5- or 6-membered) aromatic heterocyclic ring. A preferable example of the aromatic ring cluster is an aromatic ring cluster consisting of 2 or 3 aromatic rings selected from benzene, naphthalene, pyridine, pyrimidine, thiophene, furan, thiazole, isothiazole, oxazole, 1,2,4-oxadiazole, 1,3,4-oxadiazole, 1,2,4-thiadiazole, 1,3,4-thiadiazole, quinoline, isoquinoline, indole, benzothiophene, benzoxazole, benzothiazole and benzofuran. Preferable examples thereof include 2-, 3- or 4-biphenylyl, 3-(1-naphthyl)-1,2,4-oxadiazole-5-yl, 3-(2-naphthyl)-1,2,4-oxadiazole-5-yl, 3-(2-benzofuranyl)-1,2,4-oxadiazole-5-yl, 3-phenyl-1,2,4-oxadiazole-5-yl, 3-(2-benzoxazolyl)-1,2,4-oxadiazole-2-yl, 3-(3-indolyl)-1,2,4-oxadiazole-2-yl, 3-(2-indolyl)-1,2,4-oxadiazole-2-yl, 4-phenylthiazole-2-yl, 4-(2-benzofuranyl)thiazole-2-yl, 4-phenyl-1,3-oxazole-5-yl, 5-phenylisothiazole-4-yl, 5-phenyloxazole-2-yl, 4-(2-thienyl) phenyl, 4-(3-thienyl) phenyl, 3-(3-pyridyl) phenyl, 4-(3-pyridyl) phenyl, 6-phenyl-3-pyridyl, 5-phenyl-1,3,4-oxadiazole-2-yl, 4-(2-naphthyl) phenyl, 4-(2-benzofuranyl) phenyl, 4,4'-terphenyl, etc., among which biphenylyl (2-, 3- or 4-biphenylyl) is particularly preferable.

The "condensed aromatic group" refers to a monovalent group derived by removing an arbitrary hydrogen atom from a condensed polycyclic (preferably di- to tetracyclic, preferably di- or tricyclic) aromatic ring. The "condensed polycyclic aromatic ring" includes a condensed polycyclic aromatic hydrocarbon, a condensed polycyclic aromatic heterocyclic ring, etc.

The "condensed polycyclic aromatic hydrocarbon" includes, for example, C₉₋₁₄ condensed polycyclic (di- or tricyclic) aromatic hydrocarbons (e.g., naphthalene, indene, anthracene, etc.), etc.

The "condensed polycyclic aromatic heterocyclic ring" includes, for example, a 9- to 14-membered, preferably 9-or 10-membered condensed polycyclic aromatic heterocyclic ring containing one or more (preferably one to four) heteroatoms selected from nitrogen atom, sulfur atom and oxygen atom in addition to the carbon atoms. Examples thereof include aromatic heterocyclic rings such as benzofuran, benzimidazole, benzoxazole, benzothiazole, benzisothiazole, naphtho[2,3-b]thiophene, isoquinoline, quinoline, indole, quinoxaline, phenanthridine, phenothiazine, phenoxazine, phthalimide, etc.

Examples of the "condensed aromatic group" include 1-naphthyl, 2-naphthyl, 2-quinolyl, 3-quinolyl, 4-quinolyl, 2-benzofuranyl, 2-benzothiazolyl, 2-benzimidazolyl, 1-indolyl, 2-indolyl, 3-indolyl, etc., among which 1-naphthyl, 2-naphthyl etc. are preferable.

Examples of substituents on the aromatic group represented by Ar include a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, C₆₋₁₀ aryloxy-C₁₋₆ alkyl (e.g., phenoxymethyl, etc.), C₁₋₆ alkyl-C₆₋₁₀ aryl-C₂₋₆ alkenyl (e.g., methylphenylethenyl, etc.), optionally halogenated C₃₋₆ cycloalkyl, C₇₋₁₆ aralkyl which may be substituted, optionally halogenated C₁₋₆ alkoxy, optionally halogenated C₁₋₆ alkylthio, hydroxy, C₆₋₁₀ aryloxy which may be substituted, C₆₋₁₀ aryl-C₇₋₁₆ aralkyloxy (e.g., phenylbenzyloxy, etc.), amino, mono-C₁₋₆ alkylamino (e.g., methylamino, ethylamino, propylamino, isopropylamino, butylamino, etc.), di-C₁₋₆ alkylamino (e.g., dimethylamino, diethylamino, dipropylamino, dibutylamino, ethylmethylamino, etc.), 5- to 7-membered saturated cyclic amino which may be substituted, acyl, acylamino, acyloxy, etc. The "aromatic group" may have 1 to 5, preferably 1 to 3 substituents described above at substitutable positions on the aromatic group, and when the number of substituents is 2 or more, the substituents may be the same or different.

Among the substituents on the aromatic group represented by Ar, the "C₇₋₁₆ aralkyl" in the "C₇₋₁₆ aralkyl which may be substituted" includes, for example, benzyl, phenetyl, naphthylmethyl, etc.

The "C₆₋₁₀ aryloxy" in the "C₆₋₁₀ aryloxy which may be substituted" includes, for example, phenyloxy, naphthyloxy, etc. As the "substituents" on the "C₇₋₁₆ aralkyl which may be substituted" and the "C₆₋₁₀ aryloxy which may be substituted", there are used 1 to 5 substituents such as, for example, a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino (e.g., methylamino, ethylamino, propylamino, isopropylamino, butylamino, etc.), di-C₁₋₆ alkylamino (e.g., dimethylamino, diethylamino, dipropylamino, dibutylamino, ethylmethylamino, etc.), formyl, carboxy, carbamoyl, optionally halogenated C₁₋₆ alkyl-carbonyl, C₁₋₆ alkoxy-carbonyl (e.g., methoxycarbonyl, ethoxycarbonyl, propoxycarbonyl, tert-butoxycarbonyl, etc.), mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), optionally halogenated C₁₋₆ alkylsulfonyl, formylamino, optionally halogenated C₁₋₆ alkyl-carboxamide, C₁₋₆ alkoxy-carboxamide (e.g., methoxycarboxamide, ethoxycarboxamide, propoxycarboxamide, butoxycarboxamide, etc.), C₁₋₆ alkylsulfonylamino (e.g., methylsulfonylamino, ethylsulfonylamino, etc.), C₁₋₆ alkyl-carbonyloxy (e.g., acetoxy, propanoyloxy, etc.), C₁₋₆ alkoxy-carbonyloxy (e.g., methoxycarbonyloxy, ethoxycarbonyloxy, propoxycarbonyloxy, butoxycarbonyloxy, etc.), mono-C₁₋₆ alkyl-carbamoyloxy (e.g., methylcarbamoyloxy, ethylcarbamoyloxy, etc.), di-C₁₋₆ alkyl-carbamoyloxy (e.g., dimethylcarbamoyloxy, diethylcarbamoyloxy etc.), etc.

Among the substituents on the aromatic group represented by Ar, the "5- to 7-membered saturated cyclic amino" in the "5- to 7-membered saturated cyclic amino which may be substituted" includes, for example, morpholino, thiomorpholino, piperazine-1-yl, piperidino, pyrrolidine-1-yl, hexamethylene-1-yl, etc. As the "substituent" on the "5- to 7-membered saturated cyclic amino which may be substituted", there are used 1 to 3 substituents such as optionally halogenated C₁₋₆ alkyl, C₆₋₁₄ aryl which may be substituted, C₇₋₁₉ aralkyl which may be substituted, a 5- to 10-membered aromatic heterocyclic group which may be substituted, C₆₋₁₀ aryl-carbonyl which may be substituted, optionally substituted C₁₋₆ alkyl-carbonyl, optionally halogenated C₁₋₆ alkylsulfonyl, etc.

In the "C₆₋₁₄ aryl which may be substituted", the "C₆₋₁₄ aryl" includes, for example, phenyl, 1-naphthyl, 2-naphthyl, 2-indenyl, 2-anthryl, etc. The aryl is preferably phenyl or the like. In the "C₇₋₁₉ aralkyl which may be substituted", the "C₇₋₁₉ aralkyl" includes, for example, benzyl, phenetyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc., among which benzyl or the like is preferable. In the "5- to 10-membered aromatic heterocyclic ring which may be substituted", the "5- to 10-membered aromatic heterocyclic ring" includes, for example, 2-, 3- or 4-pyridyl, 1-, 2- or 3-indolyl, 2- or 3-thienyl etc., among which 2-, 3- or 4-pyridyl or the like is preferable.

In the "C₆₋₁₀ aryl-carbonyl which may be substituted", the "C₆₋₁₀ aryl-carbonyl" includes, for example, benzoyl, 1-naphthoyl, 2-naphthoyl, etc. As the "substituent" which may be possessed by the "C₆₋₁₄ aryl which may be substituted", the "C₇₋₁₉ aralkyl which may be substituted", the "5- to 10-membered aromatic heterocyclic group which may be substituted" and the "C₆₋₁₀ aryl-carbonyl which may be substituted" respectively, there are used 1 to 5 substituents such as a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino (e.g., methylamino, ethylamino, propylamino, isopropylamino, butylamino, etc.), di-C₁₋₆ alkylamino (e.g., dimethylamino, diethylamino, dipropylamino, dibutylamino, ethylmethylamino, etc.), formyl, carboxy, carbamoyl, optionally halogenated C₁₋₆ alkyl-carbonyl, C₁₋₆ alkoxy-carbonyl (e.g., methoxycarbonyl, ethoxycarbonyl, propoxycarbonyl, tert-butoxycarbonyl, etc.), mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), optionally halogenated C₁₋₆ alkylsulfonyl, formylamino, optionally halogenated C₁₋₆ alkyl-carboxamide, C₁₋₆ alkoxy-carboxamide (e.g., methoxycarboxamide, ethoxycarboxamide, propoxycarboxamide, butoxycarboxamide, etc.), C₁₋₆ alkylsulfonylamino (e.g., methylsulfonylamino, ethylsulfonylamino, etc.), C₁₋₆ alkyl-carbonyloxy (e.g., acetoxy, propanoyloxy, etc.), C₁₋₆ alkoxy-carbonyloxy (e.g., methoxycarbonyloxy, ethoxycarbonyloxy, propoxycarbonyloxy, butoxycarbonyloxy, etc.), mono-C₁₋₆ alkyl-carbamoyloxy (e.g., methylcarbamoyloxy, ethylcarbamoyloxy, etc.), and di-C₁₋₆ alkyl-carbamoyloxy (e.g., dimethylcarbamoyloxy, diethylcarbamoyloxy, etc.).

The "acyl", or "acyl" in "acylamino" and "acyloxy", as the substituent on the "aromatic group which may be substituted" represented by Ar includes, for example, acyl groups represented by the formula:

-CO-R³, -CO-OR³, -CO-NR³R⁴, -CS-NHR³, -SO₂-R^{3a} or -SO-R^{3a}

wherein R³ represents:
(i) hydrogen atom,
(ii) a hydrocarbon group which may be substituted, specifically a hydrocarbon group which may have 1 to 5 substituents selected from a halogen atom, C₁₋₃ alkylene dioxy, nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino, di-C₁₋₆ alkylamino, 5- to 7-membered cyclic amino which may be substituted, formyl, carboxy, carbamoyl, optionally halogenated C₁₋₆ alkyl-carbonyl, C₁₋₆ alkoxy-carbonyl, C₆₋₁₀ aryl-carbonyl, C₆₋₁₀ aryloxy-carbonyl, C₇₋₁₆ aralkyloxy-carbonyl, mono-C₁₋₆ alkyl-carbamoyl, di-C₁₋₆ alkyl-carbamoyl, C₆₋₁₀ aryl-carbamoyl, optionally halogenated C₁₋₆ alkylsulfonyl, C₆₋₁₀ arylsulfonyl, formylamino, optionally halogenated C₁₋₆ alkyl-carboxamide, C₆₋₁₀ aryl-carboxamide, C₁₋₆ alkoxy-carboxamide, C₁₋₆ alkylsulfonylamino, C₁₋₆ alkyl-carbonyloxy, C₆₋₁₀ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₀ aryl-carbamoyloxy, nicotinoyloxy and C₆₋₁₀ aryloxy, or
(iii) a heterocyclic group which may be substituted, specifically a heterocyclic group which may have 1 to 5 substituents selected from a halogen atom, C₁₋₃ alkylene dioxy, nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino, di-C₁₋₆ alkylamino, 5- to 7-membered cyclic amino which may be substituted, formyl, carboxy, carbamoyl, optionally halogenated C₁₋₆ alkyl-carbonyl, C₁₋₆ alkoxy-carbonyl, C₆₋₁₀ aryl-carbonyl, C₆₋₁₀ aryloxy-carbonyl, C₇₋₁₆ aralkyloxy-carbonyl, mono-C₁₋₆ alkyl-carbamoyl, di-C₁₋₆ alkyl-carbamoyl, C₆₋₁₀ aryl-carbamoyl, optionally halogenated C₁₋₆ alkyl sulfonyl, C₆₋₁₀ aryl sulfonyl, formylamino, optionally halogenated C₁₋₆ alkyl-carboxamide, C₆₋₁₀ aryl-carboxamide, C₁₋₆ alkoxy-carboxamide, C₁₋₆ alkylsulfonylamino, C₁₋₆ alkyl-carbonyloxy, C₆₋₁₀ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₀ aryl-carbamoyloxy, nicotinoyloxy and C₆₋₁₀ aryloxy,

R^{3a} represents:
(i) a hydrocarbon group which may be substituted, specifically a hydrocarbon group which may have 1 to 5 substituents selected from a halogen atom, C₁₋₃ alkylene dioxy, nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino, di-C₁₋₆ alkylamino, 5- to 7-membered cyclic amino which may be substituted, formyl, carboxy, carbamoyl, optionally halogenated C₁₋₆ alkyl-carbonyl, C₁₋₆ alkoxy-carbonyl, C₆₋₁₀ aryl-carbonyl, C₆₋₁₀ aryloxy-carbonyl, C₇₋₁₆ aralkyloxy-carbonyl, mono-C₁₋₆ alkyl-carbamoyl, di-C₁₋₆ alkyl-carbamoyl, C₆₋₁₀ aryl-carbamoyl, optionally halogenated C₁₋₆ alkyl sulfonyl, C₆₋₁₀ aryl sulfonyl, formylamino, optionally halogenated C₁₋₆ alkyl-carboxamide, C₆₋₁₀ aryl-carboxamide, C₁₋₆ alkoxy-carboxamide, C₁₋₆ alkylsulfonylamino, C₁₋₆ alkyl-carbonyloxy, C₆₋₁₀ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₀ aryl-carbamoyloxy, nicotinoyloxy and C₆₋₁₀ aryloxy, or
(ii) a heterocyclic group which may be substituted, specifically a heterocyclic group which may have 1 to 5 substituents selected from a halogen atom, C₁₋₃ alkylene dioxy, nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino, di-C₁₋₆ alkylamino, 5- to 7-membered cyclic amino which may be substituted, formyl, carboxy, carbamoyl, optionally halogenated C₁₋₆ alkyl-carbonyl, C₁₋₆ alkoxy-carbonyl, C₆₋₁₀ aryl-carbonyl, C₆₋₁₀ aryloxy-carbonyl, C₇₋₁₆ aralkyloxy-carbonyl, mono-C₁₋₆ alkyl-carbamoyl, di-C₁₋₆ alkyl-carbamoyl, C₆₋₁₀ aryl-carbamoyl, optionally halogenated C₁₋₆ alkyl sulfonyl, C₆₋₁₀ arylsulfonyl, formylamino, optionally halogenated C₁₋₆ alkyl-carboxamide, C₆₋₁₀ aryl-carboxamide, C₁₋₆ alkoxy-carboxamide, C₁₋₆ alkylsulfonylamino, C₁₋₆ alkyl-carbonyloxy, C₆₋₁₀ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₀ aryl-carbamoyloxy, nicotinoyloxy and C₆₋₁₀ aryloxy, and

R⁴ represents hydrogen atom or C₁₋₆ alkyl, or R³ and R⁴ may, together with their adjacent nitrogen atom, form a nitrogen-containing heterocyclic group, or the like.

The "5- to 7-membered saturated cyclic amino which may be substituted", used as the substituent on R³ and R^{3a}, is the same as described above.

As the hydrocarbon group represented by R³ and R^{3a}, a group derived by removing one hydrogen atom from a hydrocarbon compound, for example, linear or cyclic hydrocarbon groups (e.g., alkyl, alkenyl, alkynyl, cycloalkyl, aryl, aralkyl, etc.) are used. Among these, the following C₁₋₁₉ linear or cyclic hydrocarbon groups are preferable.
a) C₁₋₆ alkyl (e.g., methyl, ethyl, propyl, isopropyl, butyl, isobutyl, sec-butyl, tert-butyl, pentyl, hexyl, etc.),
b) C₂₋₆ alkenyl (e.g., vinyl, allyl, isopropenyl, 2-butenyl, etc.),
c) C₂₋₆ alkynyl (e.g., ethynyl, propargyl, 2-butynyl, etc.),
d) C₃₋₆ cycloalkyl (e.g., cyclopropyl, cyclobutyl, cyclopentyl, cyclohexyl, etc.) which may be condensed with one benzene ring,
e) C₆₋₁₄ aryl (e.g., phenyl, 1-naphthyl, 2-naphthyl, 2-indenyl, 2-anthryl etc.), preferably phenyl, and
f) C₇₋₁₉ aralkyl (e.g., benzyl, phenetyl, diphenylmethyl, triphenylmethyl, 1-naphthylmethyl, 2-naphthylmethyl, 2,2-diphenylethyl, 3-phenylpropyl, 4-phenylbutyl, 5-phenylpentyl, etc.), preferably benzyl.

The heterocyclic ring represented by R³ and R^{3a} is, for example, a monovalent group derived by removing an arbitrary hydrogen atom from: a 5- to 14-membered (mono-di- or tricyclic) heterocyclic ring containing one or two kinds of 1 to 4 (preferably 1 to 3) heteroatoms selected from nitrogen atom, sulfur atom and oxygen atom in addition to the carbon atoms, preferably (i) a 5- to 14-membered (preferably 5- to 10-membered) aromatic heterocyclic ring, (ii) a 5- to 10-membered non-aromatic heterocyclic ring or (iii) a 7- to 10-membered crosslinked heterocyclic ring, etc.

The "5- to 14-membered (preferably 5- to 10-membered) aromatic heterocyclic ring" includes, for example, aromatic heterocyclic rings such as thiophene, benzothiophene, benzofuran, benzimidazole, benzoxazole, benzothiazole, benzisothiazole, naphtho[2,3-b]thiophene, furan, phenoxathiine, pyrrole, imidazole, pyrazol, oxazole, 1,2,4-oxadiazole, 1,3,4-oxadiazole, 1,2,4-thiadiazole, 1,3,4-thiadiazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazane, phenoxazine, phthalimide, etc., as well as a ring formed by condensing these rings (preferably monocycle) with one or more (preferably one or two) aromatic rings (e.g., benzene ring, etc.).

The "5- to 10-membered non-aromatic heterocyclic group" includes, for example, pyrrolidine, imidazoline, pyrazolidine, pyrazoline, piperidine, piperazine, morpholine, thiomorpholine, etc.

The "7- to 10-membered crosslinked heterocyclic ring" includes, for example, quinuclidine, 7-azabicyclo[2.2.1]heptane, etc.

The "heterocyclic group" is preferably a 5- to 10-membered (monocyclic or bicyclic) heterocyclic group containing one or two kinds of preferably 1 to 4 heteroatoms selected from nitrogen atom, sulfur atom and oxygen atom in addition to the carbon atoms. Examples thereof include aromatic heterocyclic groups such as 2- or 3-thienyl, 2-, 3- or 4-pyridyl, 2- or 3-furyl, 2-, 3-, 4-, 5- or 8-quinolyl, 4-isoquinolyl, pyrazinyl, 2- or 4-pyrimidinyl, 3-pyrrolyl, 2-imidazolyl, 3-pyridazinyl, 3-isothiazolyl, 3-isoxazolyl, 1-indolyl, 2-indolyl, 2-isoindolynyl, etc., and non-aromatic heterocyclic groups such as 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholino, etc. Among these heterocyclic groups, for example a 5- or 6-membered heterocyclic group containing 1 to 3 heteroatoms selected from nitrogen atom, sulfur atom and oxygen atom in addition to the carbon atoms is preferable, and specifically use is made of 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholino, etc.

The "C₁₋₆ alkyl" represented by R⁴ includes, for example, methyl, ethyl, propyl, isopropyl, butyl, isobutyl, sec-butyl, tert-butyl, pentyl, hexyl, etc.

The "nitrogen-containing heterocyclic ring" formed by R³ and R⁴ together with their adjacent nitrogen atom includes, for example, a 5- to 7-membered nitrogen-containing heterocyclic ring which contains at least one nitrogen atom other than the carbon atoms and which may contain 1 to 3 heteroatoms selected from nitrogen atom, sulfur atom and oxygen atom, and examples thereof include piperidine, morpholine, thiomorpholine, piperazine, pyrrolidine, etc.

Preferable examples of the "acyl" as the "substituent" on the "aromatic group" represented by Ar include formyl, carboxy, carbamoyl, optionally halogenated C₁₋₆ alkyl-carbonyl, C₁₋₆ alkoxy-carbonyl (e.g., methoxycarbonyl, ethoxycarbonyl, propoxycarbonyl, tert-butoxycarbonyl, etc.), C₆₋₁₀ aryl-carbonyl which may be substituted, C₆₋₁₀ aryloxy-carbonyl which may be substituted, C₇₋₁₆ aralkyloxy-carbonyl which may be substituted, 5- to 6-membered heterocyclic carbonyl which may be substituted, mono-C₁₋₆ alkyl-carbamoyl, di-C₁₋₆ alkyl-carbamoyl (e.g., dimethylcarbamoyl, diethylcarbamoyl, ethylmethylcarbamoyl, etc.), C₆₋₁₀ aryl-carbamoyl which may be substituted, 5- to 6-membered heterocyclic carbamoyl which may be substituted, optionally halogenated C₁₋₆ alkylsulfonyl, C₆₋₁₀ arylsulfonyl which may be substituted, etc.

The "C₆₋₁₀ aryl-carbonyl" in the "C₆₋₁₀ aryl-carbonyl which may be substituted" among those described above includes, for example, benzoyl, 1-naphthoyl, 2-naphthoyl, etc. The "C₆₋₁₀ aryloxy-carbonyl" in the "C₆₋₁₀ aryloxy-carbonyl which may be substituted" includes, for example, phenoxycarbonyl etc. The "C₇₋₁₆ aralkyloxy-carbonyl" in the "C₇₋₁₆ aralkyloxy-carbonyl which may be substituted" includes, for example, benzyloxycarbonyl, phenethyloxycarbonyl, etc. The "5- to 6-membered heterocyclic carbonyl" in the "5- to 6-membered heterocyclic carbonyl which may be substituted" includes, for example, nicotinoyl, isonicotinoyl, 2-thenoyl, 3-thenoyl, 2-furoyl, 3-furoyl, morpholinocarbonyl, piperidinocarbonyl, 1-pyrrolidinyl carbonyl, etc. The "C₆₋₁₀ aryl-carbamoyl" in the "C₆₋₁₀ aryl-carbamoyl which may be substituted" includes, for example, phenyl carbamoyl, 1-naphthyl carbamoyl, 2-naphthyl carbamoyl, etc. The "5- to 6-membered heterocyclic carbamoyl" in the "5- to 6-membered heterocyclic carbamoyl which may be substituted" includes, for example, 2-pyridylcarbamoyl, 3-pyridylcarbamoyl, 4-pyridylcarbamoyl, 2-thienylcarbamoyl, 3-thienylcarbamoyl, etc. The "C₆₋₁₀ arylsulfonyl" in the "C₆₋₁₀ arylsulfonyl which may be substituted" includes, for example, benzenesulfonyl, 1-naphthalenesulfonyl, 2-naphthalenesulfonyl, etc.

As the substituent on the "C₆₋₁₀ aryl-carbonyl which may be substituted", the "C₆₋₁₀ aryloxy-carbonyl which may be substituted", the "C₇₋₁₆ aralkyloxy-carbonyl which may be substituted", the "5- to 6-membered heterocyclic carbonyl which may be substituted", the "C₆₋₁₀ aryl-carbamoyl which may be substituted", the "5- to 6-membered heterocyclic carbamoyl which may be substituted" and the "C₆₋₁₀ arylsulfonyl which may be substituted", there are used 1 to 5 substituents, preferably 1 to 3 substituents selected from a halogen atom, C₁₋₃ alkylene dioxy, nitro, cyano, optionally halogenated C₁₋₆ alkyl, optionally halogenated C₁₋₆ alkoxy, optionally halogenated C₁₋₆ alkylthio, hydroxy, amino, mono-C₁₋₆ alkylamino, di-C₁₋₆ alkylamino, formyl, carboxy, carbamoyl, optionally halogenated C₁₋₆ alkyl-carbonyl, C₁₋₆ alkoxy-carbonyl, mono-C₁₋₆ alkyl-carbamoyl, di-C₁₋₆ alkyl-carbamoyl, optionally halogenated C₁₋₆ alkylsulfonyl, formylamino, optionally halogenated C₁₋₆ alkyl-carboxamide, C₁₋₆ alkoxy-carboxamide, C₁₋₆ alkylsulfonylamino, C₁₋₆ alkyl-carbonyloxy, C₁₋₆ alkoxy-carbonyloxy, mono-C₁₋₆ alkyl-carbamoyloxy and di-C₁₋₆ alkyl-carbamoyloxy.

The "acylamino" as the "substituent" on the "aromatic group which may be substituted" represented by the above-described Ar includes, for example, an amino group substituted with 1 or 2 "acyl" groups exemplified as the "substituent" on the "aromatic group which may be substituted" represented by Ar, preferably an acylamino group represented by the formula:

-NR⁵-COR⁶, -NR⁵-COOR^{6a}, -NR⁵-SO₂RR^{6a} or -NR⁵-CONR^{6a}R^{6b}

wherein R⁵ represents hydrogen atom or C₁₋₆ alkyl, R⁶ has the same meaning as that of the above-mentioned R³, R^{6a} is as has the same meaning as that of the above-mentioned R^{3a}, and R^{6b} has the same meaning as that of the above-mentioned R⁴.

The "C₁₋₆ alkyl" represented by R⁵ and R^{6b} includes the same groups as defined with respect to the "C₁₋₆ alkyl" groups represented by R⁴.

The "acylamino" as the "substituent" on the "aromatic group which may be substituted" represented by Ar is preferably formylamino, optionally halogenated C₁₋₆ alkyl-carboxamide, C₆₋₁₀ aryl-carboxamide which may be substituted (e.g., phenylcarboxamide, naphthylcarboxamide, etc.), C₁₋₆ alkoxy-carboxamide (e.g., methoxycarboxamide, ethoxycarboxamide, propoxycarboxamide, butoxycarboxamide, etc.), C₁₋₆ alkylsulfonylamino (e.g., methylsulfonylamino, ethylsulfonylamino, etc.), etc.

The "acyloxy" as the "substituent" on the "aromatic group which may be substituted" represented by the above-described Ar includes, for example, oxy substituted with one "acyl" group exemplified as the "substituent" on the above-mentioned "aromatic group which may be substituted", preferably those acyloxy groups represented by the formula:

-O-COR⁷, -O-COOR⁷ or -O-CONHR⁷

wherein R⁷ has the same meaning as that of the above-mentioned R³.

The "acyloxy" as the "substituent" on the "aromatic group which may be substituted" represented by Ar is preferably C₁₋₆ alkyl-carbonyloxy (e.g., acetoxy, propanoyloxy, etc.), C₆₋₁₀ aryl-carbonyloxy which may be substituted (e.g., benzoyloxy, 1-naphthoyloxy, 2-naphthoyloxy, etc.), C₁₋₆ alkoxy-carbonyloxy (e.g., methoxycarbonyloxy, ethoxycarbonyloxy, propoxycarbonyloxy, butoxycarbonyloxy, etc.), mono-C₁₋₆ alkyl-carbamoyloxy (e.g., methylcarbamoyloxy, ethylcarbamoyloxy, etc.), di-C₁₋₆ alkyl-carbamoyloxy (e.g., dimethylcarbamoyloxy, diethylcarbamoyloxy, etc.), C₆₋₁₀ aryl-carbamoyloxy which may be substituted (e.g., phenylcarbamoyloxy, naphthylcarbamoyloxy, etc.), nicotinoyloxy, etc. In the "C₆₋₁₀ aryl-carboxamide which may be substituted", the "C₆₋₁₀ aryl-carbonyloxy which may be substituted" and the "C₆₋₁₀ aryl-carbamoyloxy which may be substituted", the "substituent" and "preferable examples" thereof are the same "substituent" on the above-described "C₆₋₁₀ aryl-carbony which may be substituted".

Among those described above, Ar is preferably a ring-assembled aromatic group which may be substituted (particularly biphenyl such as 2-, 3- or 4-biphenyl, etc.).

In the formulas, X represents a divalent group selected from -O-, -S-, -CO-, -SO-, -SO₂-, -NR⁸-, -CONR⁸- (including -CONR⁸- and -NR⁸CO-), -SO₂NR⁸- (including -SO₂NR⁸- and -NR⁸SO₂-) and -COO- (R⁸ represents hydrogen atom, a hydrocarbon group which may be substituted, or acyl which may be substituted), a divalent C₁₋₆ aliphatic hydrocarbon group which may contain one or two of these divalent groups, or a linking bond, Y represents a divalent group selected from -O-, -S-, -CO-, -SO-, -SO₂-, -NR⁸-, -CONR⁸- (including -CONR⁸- and -NR⁸CO-), -SO₂NR⁸- (including -SO₂NR⁸- and -NR⁸SO₂-) and -COO- (R⁸ represents hydrogen atom, a hydrocarbon group which may be substituted, or acyl which may be substituted) or a divalent C₁₋₆ aliphatic hydrocarbon group which may contain one or two of these divalent groups.

The hydrocarbon group which may be substituted, represented by R⁸, includes the same "hydrocarbon group which may be substituted" represented by the above-mentioned R³. In particular, optionally halogenated C₁₋₆ alkyl or the like is preferable.

The acyl represented by R⁸ includes the same "acyl" as the substituent on the aromatic group represented by the above-mentioned Ar. Particularly preferable examples thereof include formyl, carbamoyl, optionally halogenated C₁₋₆ alkyl-carbonyl, C₁₋₆ alkoxy-carbonyl (e.g., methoxycarbonyl, ethoxycarbonyl, propoxycarbonyl, tert-butoxycarbonyl, etc.), C₆₋₁₀ aryl-carbamoyl which may have the above-described substituent, C₆₋₁₀ aryloxy-carbamoyl which may have the above-described substituent, C₇₋₁₆ aralkyloxy-carbamoyl which may have the above-described substituent, 5- or 6-membered heterocyclic carbonyl which may have the above-described substituent, mono-C₁₋₆ alkyl-carbamoyl, di-C₁₋₆ alkyl-carbamoyl (e.g., dimethylcarbamoyl, diethylcarbamoyl, ethylmethylcarbamoyl, etc.), C₆₋₁₀ aryl-carbamoyl which may have the above-described substituent, 5- or 6-membered heterocyclic carbamoyl which may have the above-described substituent, optionally halogenated C₁₋₆ alkylsulfonyl, C₆₋₁₀ arylsulfonyl which may have the above-described substituent, etc., particularly preferably optionally halogenated C₁₋₆ alkyl-carbonyl.

The C₁₋₆ aliphatic hydrocarbon group includes, for example, C₁₋₆ alkylene, C₂₋₆ alkenylene, C₂₋₆ alkynylene, etc.

The C₁₋₆ alkylene includes, for example, not only linear C₁₋₆ alkylene such as -CH₂-, -(CH₂)₂-, -(CH₂)₃-, -(CH₂)₄-, -(CH₂)₅-, -(CH₂)₆-, etc., but also C₁₋₃ alkylene which may have one to three C₁₋₃ alkyl groups (for example, -CH₂-, -(CH₂)₂-, -(CH₂)₃-, etc.), etc.

The C₂₋₆ alkenylene includes, for example, not only linear C₂₋₆ alkenylene such as -CH=CH-, -CH₂-CH=CH-, etc., but also C₂₋₃ alkenylene which may have one to three C₁₋₃ alkyl groups (for example, -CH=CH-, -CH₂-CH=CH-, etc.), etc.

The C₂₋₆ alkynylene includes, for example, not only linear C₂₋₆ alkynylene such as -C≡C-, -CH₂-C≡C-, -C≡C-CH₂-, -C≡C-CH₂CH₂-, -CH₂CH₂-C≡C-, -CH₂-C≡C-CH₂-, -(CH₂)₂-C≡C-CH₂-, (CH₂)₂-C≡C-(CH₂)₂-, -(CH₂)₃-C≡C-CH₂-, etc., but also C₂₋₃ alkynylene which may have one to three C₁₋₃ alkyl groups (for example, -C≡C-, -CH₂-C≡C-, -C≡C-CH₂-, -C≡C-CH₂CH₂-, -CH₂CH₂-C≡C-, etc.) .

The C₁₋₆ aliphatic hydrocarbon group is particularly preferably C₁₋₃ aliphatic hydrocarbon group such as C₁₋₃ alkylene, C₂₋₃ alkenylene, C₂₋₆ alkynylene, etc.

The divalent C₁₋₆ aliphatic hydrocarbon group containing one or two divalent groups selected from -O-, -S-, -CO-, -SO-, -SO₂-, -NR⁸-, -CONR⁸-, -SO₂NR⁸- and -COO- , represented by X, includes for example:
(i) -(CH₂)_{w}O-, -(CH₂)_{w}S-, -(CH₂)_{w}CO-, -(CH₂)_{w}SO-, -(CH₂)_{w}SO₂-, -(CH₂)_{w}NR⁸-, -(CH₂)_{w}CONR⁸-, -(CH₂)_{w}NR⁸CO-, -(CH₂)_{w}SO₂NR⁸-, -(CH₂)_{w}NR⁸SO₂-, -(CH₂)_{w}COO-,
(ii) -O(CH₂)_{w}-, -S(CH₂)_{w}-, -CO(CH₂)_{w}-, -SO(CH₂)_{w}-, -SO₂(CH₂)_{w}-, -NR⁸(CH₂)_{w}-, -CONR⁸(CH₂)_{w}-, -NR⁸CO(CH₂)_{w}-, -SO₂NR⁸(CH₂)_{w}-, -NR⁸SO₂(CH₂)_{w}-, -COO(CH₂)_{w}-, and
(iii) -(CH₂)_{w1}O(CH₂)_{w2}-, -(CH₂)_{w1}S(CH₂)_{w2}-, -(CH₂)_{w1}CO(CH₂)_{w2}-, -(CH₂)_{w1}SO(CH₂)_{w2}-, -(CH₂)_{w1}SO₂(CH₂)_{w2}-, -(CH₂)_{w1}NR⁸(CH₂)_{w2}-, (CH₂)_{w1}CONR⁸(CH₂)_{w2}-, -(CH₂)_{w1}NR⁸CO(CH₂)_{w2}-, -(CH₂)_{w1}SO₂NR⁸(CH₂)_{w1}-, -(CH₂)_{w1}NR⁸SO₂(CH₂)_{w1}-, -(CH₂)_{w1}COO(CH₂)_{w1}-, and the like.

The divalent C₁₋₆ aliphatic hydrocarbon group containing one or two divalent groups selected from -O-, -S-, -CO-, -SO-, -SO₂-, -NR⁸-, -CONR⁸-, -SO₂NR⁸- and -COO- , represented by Y, includes for example:
(i) -O(CH₂)_{w}-, -S(CH₂)_{w}-, -CO(CH₂)_{w}-, -SO(CH₂)_{w}-, -SO₂(CH₂)_{w}-, -NR⁸(CH₂)_{w}-, -CONR⁸(CH₂)_{w}-, -NR⁸CO(CH₂)_{w}-, -SO₂NR⁸(CH₂)_{w}-, -NR⁸SO₂(CH₂)_{w}-, -COO(CH₂)_{w}-, and
(ii) -(CH₂)_{w1}O(CH₂)_{w2}-, -(CH₂)_{w1}S(CH₂)_{w2}-, -(CH₂)_{w1}CO(CH₂)_{w2}-, -(CH₂)_{w1}SO(CH₂)_{w2}-, -(CH₂)_{w1}SO₂(CH₂)_{w2}-, -(CH₂)_{w1}NR⁸(CH₂)_{w2}-, -(CH₂)_{w1}CONR⁸(CH₂)_{w2}-, -(CH₂)_{w1}NR⁸CO(CH₂)_{w2}-, -(CH₂)_{w1}SO₂NR⁸(CH₂)_{w1}-, -(CH₂)_{w1}NR⁸SO₂(CH₂)_{w1}-, -(CH₂)_{w1}COO(CH₂)_{w1}-, and the like.

w is an integer of 1 to 6, preferably 1 to 4, particularly preferably 1 or 2.

Each of w1 and w2 is an integer of 1 to 3, particularly preferably 1 or 2.

Further, preferable examples of X and Y include:
C₁₋₅ alkylene, C₂₋₅ alkenylene, C₂₋₅ alkynylene, -CH₂-Z-, -(CH₂)₂-Z-, -(CH₂)₃-Z-, -(CH₂)₄-Z-, -Z-CH₂-, -Z-(CH₂)₂-, -Z-(CH₂)₃-, -Z-(CH₂)₄-, -Z-CH₂-Z-, -Z-(CH₂)₂-Z-, -Z-(CH₂)₃-Z-, -CH₂-Z-CH₂-, -(CH₂)₂-Z-CH₂-, -(CH₂)₃-Z-CH₂-, -CH₂-Z-(CH₂)₂- or -CH₂-Z-(CH₂)₃-
wherein Z represents -O-, -S-, -CO-, -SO-, -SO₂-, -NR⁸-, -CONR⁸-, -SO₂NR⁸- or -COO-, and when two Z groups are present in the same formula, they are the same or different.

In those described above, preferable examples of X include -(CH₂)pO- (p is an integer of 1 to 3), -CONH-, -SO₂NH- or C₁₋₃ alkylene, particularly preferably -(CH₂)pO- (p is an integer of 1 to 3).

Preferable examples of Y include C₁₋₃ alkylene, -(CH₂)qCONR⁹(CH₂)r- (each of q and r is 0 to 3, and the sum of q and r is an integer of 3 or less, R⁹ represents a hydrogen atom or optionally halogenated C₁₋₆ alkyl or optionally halogenated C₁₋₆ alkyl-carbonyl) or -(CH₂)qCOO(CH₂)r- (each symbol is as defined above), particularly preferably C₁₋₃ alkylene or -(CH₂)qCONR⁹(CH₂)r- (each symbol is as defined above).

The hydrocarbon group which may be substituted, represented by R¹ or R², includes the same "hydrocarbon group which may be substituted" represented by the above-described R³. In the "hydrocarbon group which may be substituted", the "hydrocarbon group" is particularly preferably C₁₋₆ alkyl. Preferable examples of the C₁₋₆ alkyl include methyl, ethyl, propyl, isopropyl, butyl, isobutyl, sec-butyl, tert-butyl, pentyl, hexyl, etc., among which methyl, ethyl and propyl are particularly preferable.

As the substituent on the hydrocarbon group which may be substituted, represented by R¹ or R², there are used 1 to 5 substituents, preferably 1 to 3 substituents such as, a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, optionally halogenated C₃₋₆ cycloalkyl, optionally halogenated C₁₋₆ alkoxy, optionally halogenated C₁₋₆ alkylthio, hydroxy, amino, mono-C₁₋₆ alkylamino (e.g., methylamino, ethylamino, propylamino, isopropylamino, butylamino, etc.), di-C₁₋₆ alkylamino (e.g., dimethylamino, diethylamino, dipropylamino, dibutylamino, ethylmethylamino, etc.), formyl, carboxy, carbamoyl, optionally halogenated C₁₋₆ alkyl-carbonyl, C₁₋₆ alkoxy-carbonyl (e.g., methoxycarbonyl, ethoxycarbonyl, propoxycarbonyl, tert-butoxycarbonyl, etc.), mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), optionally halogenated C₁₋₆ alkylsulfonyl, formylamino, optionally halogenated C₁₋₆ alkyl-carboxamide, C₁₋₆ alkoxy-carboxamide (e.g., methoxycarboxamide, ethoxycarboxamide, propoxycarboxamide, butoxycarboxamide, etc.), C₁₋₆ alkylsulfonylamino (e.g., methylsulfonylamino, ethylsulfonylamino, etc.), C₁₋₆ alkyl-carbonyloxy (e.g., acetoxy, propanoyloxy, etc.), C₁₋₆ alkoxy-carbonyloxy (e.g., methoxycarbonyloxy, ethoxycarbonyloxy, propoxycarbonyloxy, butoxycarbonyloxy, etc.), mono-C₁₋₆ alkyl-carbamoyloxy (e.g., methylcarbamoyloxy, ethylcarbamoyloxy, etc.), di-C₁₋₆ alkyl-carbamoyloxy (e.g., dimethylcarbamoyloxy, diethylcarbamoyloxy, etc.), an aromatic group which may be substituted, etc. When the number of substituents is 2 or more, the substituents may be the same or different.

The hydrocarbon group which may be substituted, represented by R¹ or R², is particularly preferably optionally halogenated C₁₋₆ alkyl or the like.

In the "nitrogen-containing heterocyclic ring which may contain a substituent" formed by R¹ and R² together with their adjacent nitrogen atom, the "nitrogen-containing heterocyclic ring" is, for example, a 3- to 8-membered nitrogen-containing heterocyclic ring which contains at least one nitrogen atom in addition to the carbon atoms and which may contain one to three heteroatoms selected from nitrogen atom, sulfur atom and oxygen atom. Examples thereof include aziridine, azetidine, morpholine, thiomorpholine, piperidine, piperazine, pyrrolidine, hexamethylene imine, heptamethylene imine, hexahydropyrimidine, 1,4-diazepam, unsaturated cyclic amines thereof (e.g., 1,2,5,6-tetrahydropyridine, etc.), etc., among which morpholine, piperidine, piperazine and pyrrolidine are preferable.

As the "substituent" on the "nitrogen-containing heterocyclic ring which may be substituted" formed by R¹ and R² together with their adjacent nitrogen atom, there are used, for example, the same 1 to 3 substituents as those which may be possessed by the above-described "5- to 7-membered saturated cyclic amino which may be substituted".

Preferable examples of R¹ and R² include C₁₋₆ alkyl (e.g., methyl, ethyl, propyl, etc.), and it is preferable that R¹ and R² together with their adjacent nitrogen atom form piperidino, pyrrolidine-1-yl, etc.

It is preferable that at least one of R¹ and R² represents C₁₋₆ alkyl which may be substituted, and it is particularly preferable that both R¹ and R² represent C₁₋₆ alkyl which may be substituted,

As the ring represented by ring A in the above formula, for example, an aromatic ring such as monocyclic aromatic ring or condensed aromatic ring is used. The ring A is substituted at any possible positions with a group represented by the formula Ar-X- and a group represented by the formula: 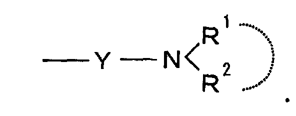 The ring A may further have substituent(s) in addition to the group represented by the formula Ar-X- and the group represented by the formula: 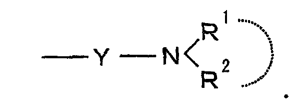 As the substituent, for example a halogen atom (e.g., fluorine, chlorine, bromine, iodine, etc.), optionally halogenated C₁₋₆ alkyl, optionally halogenated C₁₋₆ alkoxy, hydroxy, amino, etc. are used. The "optionally halogenated C₁₋₆ alkyl" and "optionally halogenated C₁₋₆ alkoxy" are the same as the "optionally halogenated C₁₋₆ alkyl" and "optionally halogenated C₁₋₆ alkoxy" described in detail in the above-mentioned Ar. The substituent on the ring A is particularly preferably a halogen atom (e.g., chlorine, etc.), C₁₋₆ alkoxy (e.g., methoxy, etc.), etc. The ring A may be substituted with 1 to 3 of these substituents at any possible positions, and when the number of substituents is 2 or more, the substituents may be the same or different. The ring A is substituted particularly preferably with only a group represented by the formula Ar-X- and a group represented by the formula:

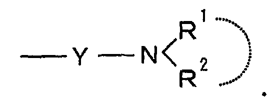

As the "monocyclic aromatic ring", for example a benzene ring or a 5- or 6-membered aromatic heterocyclic ring is used. The "5- or 6-membered aromatic heterocyclic ring" includes, for example, a 5- or 6-membered aromatic heterocyclic ring containing one or more (for example 1 to 3, preferably 1 to 2) heteroatoms selected from nitrogen atom, sulfur atom and oxygen atom in addition to the carbon atoms. Specifically, there are used thiophene, furan, pyrrole, imidazole, pyrazol, thiazole, oxazole, pyridine, 2-pyridone, pyrazine, pyrimidine, pyridazine, etc.

The "monocyclic aromatic ring" is preferably benzene, pyridine or 2-pyridone.

The "monocyclic aromatic ring" may be substituted with the same substituents as those on the above-described ring A.

As the "condensed aromatic ring", there are used a ring represented by the formula: 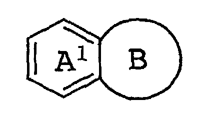 wherein the ring A¹ represents a benzene ring which may further be substituted, and the ring B represents a 4- to 8-membered ring which may further be substituted.

As the substituent on the benzene ring represented by the ring A¹, the same substituent as that on the above-mentioned ring A is used.

The ring A¹ is preferably a benzene ring substituted with only the group represented by the formula Ar-X-.

The 4- to 8-membered ring represented by the ring B is a 4- to 8-membered homo- or heterocyclic ring which may contain one double bond in a portion other than the portion condensed with the ring A¹ and which may contain 1 to 3 heteroatoms selected from oxygen atom, nitrogen atom and sulfur atom in addition to the carbon atoms. Examples thereof include rings represented by the formula: 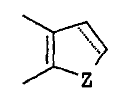 wherein --- line represents a single or double bond, and Z represents (i) a bond, (ii) C₁₋₄ alkylene, (iii) C₂₋₄ alkenylene, (iv) -O-CH₂-, (v) -O-CH₂-CH₂- or (vi) the formula -NR⁸-CH₂- or -NR⁸-CH₂-CH₂- wherein R⁸ represents hydrogen atom, or a hydrocarbon group or acyl which may be substituted. R⁸ is as defined above, and preferable examples thereof include hydrogen atom, optionally halogenated C₁₋₆ alkyl (e.g., C₁₋₆ alkyl which may have 1 to 5 halogen atoms described above, etc.), C₁₋₆ alkyl-carbonyl (e.g., acetyl, propionyl, etc.), C₁₋₆ alkoxy-carbonyl (e.g., methoxycarbonyl, ethoxycarbonyl, propoxycarbonyl, tert-butoxycarbonyl, etc.), C₆₋₁₀ aryl-carbonyl (e.g., benzoyl, 1-naphthoyl, 2-naphthoyl, etc.), C₆₋₁₀ aryloxy-carbonyl (e.g., phenoxycarbonyl, etc.), C₇₋₁₆ aralkyloxy-carbonyl (e.g., benzyloxycarbonyl, phenetyloxycarbonyl, etc.), 5- to 6-membered heterocyclic carbonyl (e.g., nicotinoyl, isonicotinoyl, 2-thenoyl, 3-thenoyl, 2-furoyl, 3-furoyl, morpholinocarbonyl, piperidinocarbonyl, 1-pyrrolidinyl carbonyl, etc.), mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), C₆₋₁₀ aryl-carbamoyl (e.g., phenylcarbamoyl, 1-naphthylcarbamoyl, 2-naphthylcarbamoyl, etc.), 5- to 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl, etc.), C₁₋₆ alkylsulfonyl (e.g., methylsulfonyl, ethylsulfonyl, etc.) and C₆₋₁₀ arylsulfonyl (e.g., benzenesulfonyl, 1-naphthalenesulfonyl, 2-naphthalenesulfonyl, etc.). R⁸ is more preferably a hydrogen atom, optionally halogenated C₁₋₆ alkyl, C₁₋₆ alkyl-sulfonyl and C₁₋₃ alkylsulfonyl.]

Z is preferably C₁₋₃ alkylene, -NR⁸-CH₂-, etc. Z is more preferably ethylene.

The "4- to 8-membered ring" is preferably a ring represented by the formula: 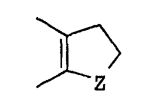 wherein Z is as defined above. The 4- to 8-membered ring is preferably a 6-membered homo- or heterocyclic ring which does not contain a double bond in the other portion than the portion condensed with the ring A¹ and which may contain one oxygen atom or imino in addition to the carbon atoms.

In the "4- to 8-membered ring which may be substituted" represented by the ring B, the "substituent" includes, for example, oxo, C₁₋₆ alkyl (e.g., methyl, ethyl, propyl, isopropyl, butyl, etc.), hydroxy, etc. The ring B may be substituted with 1 to 3 substituents at any possible positions, and when the number of substituents is 2 or more, the substituents may be the same or different.

The ring B is preferably a 6-membered homo- or heterocyclic ring not having a substituent except for a group represented by:

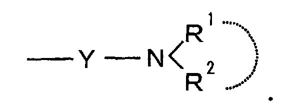

The condensed ring formed by the rings A¹ and B is preferably a ring represented by the formula:

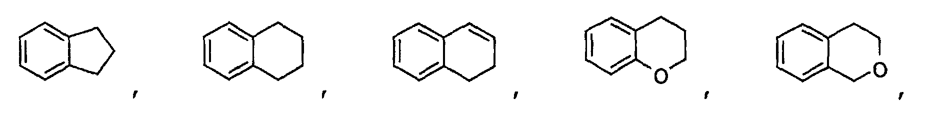

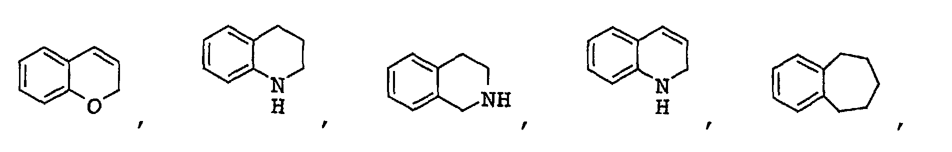

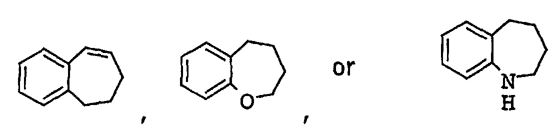

The ring A is preferably benzene ring, a 6-membered nitrogen-containing aromatic heterocyclic ring (particularly, pyridine ring, 2-pyridone ring, etc.) or tetralin ring, each of which may be substituted with a halogen atom (particularly chlorine, etc.) and/or C₁₋₆ alkoxy (particularly methoxy, etc.). The ring A is more preferably benzene ring or tetralin ring. The ring A is still more preferably a tetralin ring which is not substituted with a group except for a group represented by the formula Ar-X- and a group represented by the formula: 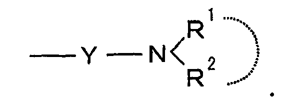 The ring A is most preferably tetralin ring not having a substituent at the 1- or 4-position on the ring.

Compound (I) is preferably a compound wherein examples of the respective symbols described above are arbitrarily combined, particularly preferably a compound wherein Ar is biphenylyl, X is -(CH₂)pO- (p is an integer of 1 to 3), -CONH-, -SO₂NH- or C₁₋₃ alkylene (particularly -(CH₂)pO-), Y is C₁₋₃ alkylene, -(CH₂)qCONH(CH₂)r- (each of q and r is 0 to 3, and the sum of q and r is an integer of 3 or less) or -(CH₂)qCOO(CH₂)r- (each symbol has the same meaning as defined above) (particularly C₁₋₃ alkylene or -(CH₂)qCONH(CH₂)r-), and R¹ and R² each represent a hydrogen atom or C₁₋₆ alkyl (particularly C₁₋₃ alkyl such as methyl, ethyl and propyl), or R¹ and R² together with their adjacent nitrogen atom form a 5- or 6-membered nitrogen-containing heterocyclic ring (particularly piperidino, pyrrolidine-1-yl, etc.), and the ring A is benzene ring, a 6-membered nitrogen-containing aromatic heterocyclic ring (particularly pyridine ring, 2-pyridone ring, etc.) or tetralin ring (particularly benzene ring or tetralin ring), each of which may substituted with a halogen atom (particularly chlorine, etc.) and/or C₁₋₆ alkoxy (particularly methoxy, etc).

Compound (I) is particularly preferably (R)-(+)-6-(4-biphenylyl)methoxy-2-[2-(N,N-dimethylamino)ethyl]tetralin hydrochloride·1H₂O, 4-(4-biphenylylmethoxy) phenyl-N-[2-(N, N-dimethylamino)ethyl]acetamide or the like.

Among Compound (I) described above, Compound (I') described above is preferred, particularly, a compound represented by the formula (Ia): 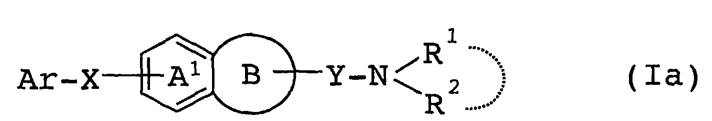 wherein the ring A¹ represents a monocyclic aromatic ring which may further be substituted, and the other symbols are as defined above, or a salt thereof, or a compound represented by the formula (Ib): 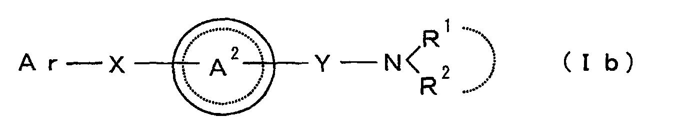 wherein the symbols are as defined above, or a salt thereof, is preferred.

As the "monocyclic aromatic ring which may further be substituted" represented by the ring A¹, the monocyclic aromatic ring and substituents exemplified as those of the above-described ring A are used.

In Compound (Ia), it is preferable that Ar represents biphenylyl, X represents -(CH₂)pO- (p is an integer of 1 to 3), Y represents C₁₋₃ alkylene, R¹ and R² each represent a hydrogen atom or C₁₋₆ alkyl (particularly C₁₋₃ alkyl such as methyl, ethyl and propyl), or R¹ and R² together with their adjacent nitrogen atom form a 5- or 6-membered nitrogen-containing heterocyclic ring (particularly piperidino, pyrrolidine-1-yl, etc.), and the ring A, that is, a condensed ring consisting of the rings A¹ and B, is a tetralin ring which may be substituted with a halogen atom (particularly chlorine, etc.) and/or C₁₋₆ alkoxy (particularly methoxy, etc.).

In Compound (Ib), it is preferable that Ar represents biphenylyl, X represents -(CH₂)pO- (p is an integer of 1 to 3), Y represents -(CH₂)_{q}CONH(CH₂)r- (each of q and r is 0 to 3 and the sum of q and r is an integer of 3 or less), R¹ and R² each represent a hydrogen atom or C₁₋₆ alkyl (particularly C₁₋₃ alkyl such as methyl, ethyl, propyl, etc.), or R¹ and R² together with their adjacent nitrogen atom form a 5- or 6-membered nitrogen-containing heterocyclic ring (particularly piperidino, pyrrolidine-1-yl etc.), and the ring A² is a benzene ring or a 6-membered nitrogen-containing aromatic heterocyclic ring (particularly pyridine ring, 2-pyridone ring, etc.), each of which may be substituted with a halogen atom (particularly chlorine, etc.) and/or C₁₋₆ alkoxy (particularly methoxy, etc.).

Salts of Compounds (I), (I'), (Ia) and (Ib) include, for example, a salt with an inorganic base, an ammonium salt, a salt with an organic base, a salt with an inorganic acid, a salt with an organic acid, and a salt with a basic or acidic amino acid.

Preferable examples of the salt with an inorganic base include alkali metal salts such as sodium salt, potassium salt etc.; alkaline earth metal salts such as calcium salt, magnesium salt, barium salt etc.; and aluminum salts. Preferable examples of the salt with an organic base include salts with trimethylamine, triethylamine, pyridine, picoline, ethanolamine, diethanolamine, triethanolamine, dicyclohexylamine, N,N-dibenzylethylenediamine, etc. Preferable examples of the salt with an inorganic acid include salts with hydrochloric acid, hydrobromic acid, nitric acid, sulfuric acid, phosphoric acid, etc. Preferable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. Preferable examples of the salt with a basic amino acid include salts with arginine, lysine, ornithine etc., and preferable examples of the salt with an acidic amino acid include salts with aspartic acid, glutamic acid, etc.

Among these salts, pharmaceutically acceptable salts are preferable. For example, when an acidic functional group is present in Compounds (I), (I'), (Ia) or (Ib), inorganic salts such as alkali metal salts (for example, sodium salt, potassium salt, etc.) or alkaline earth metal salts (for example, calcium salt, magnesium salt, barium salt etc.), or ammonium salts are used, and when a basic functional group is present in Compounds (I), (Ia) or (Ib), inorganic salts such as hydrochloride, sulfate, phosphate and hydrobromate or organic salts such as acetate, maleate, fumarate, succinate, methanesulfonate, p-toluenesulfonate, citrate and tartrate are used.

Compounds (I), (I'), (Ia) or (Ib) may be an anhydride or hydrate. The hydrate may have one to three H₂O molecules.

Compound (I), (I'), (Ia) or (Ib) may be prodrugs thereof. The prodrugs of Compound (I), (I'), (Ia) or (Ib) are those compounds converted into Compound (I), (I'), (Ia) or (Ib) by reaction with an enzyme or stomach acid etc. under physiological conditions in the living body, that is, (a) those compounds converted into Compound (I), (I'), (Ia) or (Ib) by enzymatic oxidation, reduction, hydrolysis etc. and (b) those compounds converted into Compound (I), (I'), (Ia) or (Ib) by hydrolysis with stomach acid, etc. The prodrugs of Compound (I), (I'), (Ia) or (Ib) are those compounds (or salts thereof) wherein a hydroxyl group of Compound (I), (I'), (Ia) or (Ib) is acylated, alkylated, phosphorylated or borated (for example, those compounds wherein a hydroxyl group of Compound (I), (I'), (Ia) or (Ib) is acetylated, palmitoylated, propanoylated, pivaloylated, succinylaed, fumarylated, alanylated or dimethylaminomethylcarbonylated, or salts thereof) or those compounds wherein a carboxyl group of Compound (I), (I'), (Ia) or (Ib) is esterified or amidated (for example, those compounds wherein a carboxyl group of Compound (I), (I'), (Ia) or (Ib) is ethylesterified, phenylesterified, carboxyoxymethylesterified, dimethylaminomethylesterified, pivaloyloxymethylesterified, ethoxycarbonyloxyethylesterified, phthalidylesterified, (5-methyl-2-oxo-1,3-dioxolane-4-yl)methylesterified, cyclohexyloxycarbonylethylesterified or methylamidated, or salts thereof). These prodrugs can be produced by a process known per se or its analogous process.

Further, the prodrugs of Compound (I), (I'), (Ia) or (Ib) may be those converted into Compounds (I), (I'), (Ia) or (Ib) under those physiological conditions described in "Iyakuhin No Kaihatsu" (Development of Pharmaceutical Preparations), Vol. 7, Molecular Design, pp. 163-198, published in 1990 by Hirokawa Shoten.

Compound (I), (I'), (Ia) or (Ib) may be labeled with an isotope (e.g., ²H, ³H, ¹⁴C, ³⁵S, ¹²⁵I etc.).

Compounds (I) and (I') can be produced according to the production process described in JP 11-80098 A or the following process for producing Compound (Ib), or can be produced by a process known per se or its analogous process. Compound (Ia) can also be produced according to the production process described in JP 11-80098 A, or when an amide linkage and an ether linkage are present in the same molecule, Compound (Ia) can be produced according to its modified method by selectively cleaving the ether linkage only in the presence of methanesulfonic acid and methionine, then subjecting the product to alkylation reaction and reducing the amide moiety.

The process for producing Compound (Ib) will be illustrated hereinafter.

Compound (Ib) wherein, for example, X contains oxygen atom, sulfur atom which may be oxidized or imino which may be substituted is produced according to the following production method.

Usually, the "room temperature" refers to 0 to 30°C.

Unless otherwise specified, each symbol in the chemical structures shown in the scheme below has the same meaning as defined above. 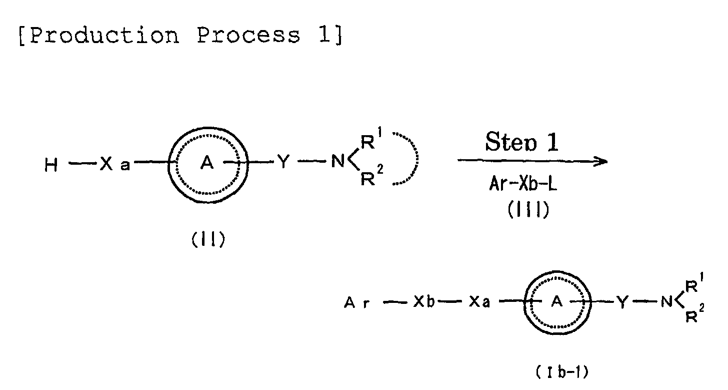 wherein Xa represents oxygen atom, sulfur atom which may be oxidized or imino which may be substituted. The "imino which may be substituted" represented by Xa is the same as the "imino which may be substituted" represented by the above-described R⁸.

### (Step 1)

Compound (II) can be subjected to an alkylation reaction or an acylation reaction to obtain Compound (Ib-1).

The "alkylation reaction" and "acylation reaction" can be carried out according to a method known per se, for example, the method described in Organic Functional Group Preparations, 2nd ed., Academic Press, Inc., 1989, etc.

Specifically, Compound (II) can be reacted with a compound represented by the formula Ar-Xb-L (III) wherein Xb represents a group derived from X by removing Xa, and L represents a leaving group or hydroxy to obtain Compound (Ib-1).

The leaving group represented by L includes a halogen atom (e.g., chlorine, bromine, iodine, etc.), optionally halogenated C₁₋₆ alkylsulfonyloxy (e.g., methanesulfonyloxy, ethanesulfonyloxy, trifluoromethanesulfonyloxy etc.), and C₆₋₁₀ arylsulfonyloxy which may be substituted.

As the substituent on the "C₆₋₁₀ aryl sulfonyloxy which may be substituted", there are used 1 to 3 groups selected from e.g. a halogen atom, optionally halogenated C₁₋₆ alkyl and C₁₋₆ alkoxy. Examples of the "C₆₋₁₀ arylsulfonyloxy which may be substituted" include benzenesulfonyloxy, p-toluenesulfonyloxy, 1-naphthalenesulfonyloxy and 2-naphthalenesulfonyloxy.

For example, when L is a leaving group, Compound (II) is reacted with an equal or excess amount of Compound (III) in an inert solvent. If necessary, a base may be added to the reaction. When Xa is imino which may be substituted, the base is not always necessary.

The reaction temperature is usually at about -20°C to 100°C, preferably room temperature to 80°C. The reaction time is usually about 0.5 hour to 1 day.

The inert solvent is for example an alcoholic solvent, ether solvent, halogen solvent, aromatic solvent, nitrile solvent, amide solvent, ketone solvent, sulfoxide solvent or water, which can be used alone or in a combination thereof. In particular, acetonitrile, N,N-dimethylformamide (DMF), acetone, ethanol and pyridine are preferable.

The base includes:
1) strong bases such as, for example, alkali metal or alkaline earth metal hydrides (for example, lithium hydride, sodium hydride, potassium hydride, calcium hydride, etc.), alkali metal or alkaline earth metal amides (for example, lithium amide, sodium amide, lithium diisopropyl amide, lithium dicyclohexyl amide, lithium hexamethyl disilazide, sodium hexamethyl disilazide, potassium hexamethyl disilazide, etc.), and alkali metal or alkaline earth metal lower alkoxides (for example, sodium methoxide, sodium ethoxide, potassium tert-butoxide, etc.);
2) inorganic bases such as, for example, alkali metal, alkaline earth metal or silver hydroxides (for example, silver hydroxide, sodium hydroxide, potassium hydroxide, lithium hydroxide, barium hydroxide, etc.), alkali metal, alkaline earth metal or silver carbonates (for example, sodium carbonate, potassium carbonate, cesium carbonate, silver carbonate, etc.), alkali metal or alkaline earth metal bicarbonates (for example, sodium bicarbonate, potassium bicarbonate, etc.), and silver oxide; and
3) organic bases, for example, amines such as triethylamine, diisopropylethylamine, N-methylmorpholine, dimethylaminopyridine, DBU (1,8-diazabicyclo[5.4.0]undece-7-ene), DBN (1,5-diazabicyclo[4.3.0]none-5-ene), etc., and basic heterocyclic compounds such as pyridine, imidazole, 2,6-lutidine, etc.

Preferred reaction conditions are such that, for example, in case of an alkylation reaction, Compound (II), 1 to 2 equivalents of Compound (III) and 1 to 5 equivalents of a base (for example, potassium carbonate, sodium hydride, sodium hydroxide, silver carbonate etc.) are stirred in acetonitrile or DMF for about 1 hour to 2 days. The preferable reaction temperature is varied depending on the base used, but usually the reaction temperature is preferably room temperature when sodium hydride is used, or room temperature to 80°C when potassium carbonate is used.

Preferred reaction conditions are such that, for example, in case of an acylation reaction, Compound (II), 1 to 1.5 equivalents of Compound (III) and 1 to 5 equivalents of a base (for example, sodium hydride, sodium hydroxide, potassium carbonate, sodium bicarbonate, triethylamine etc.) are stirred in an inert solvent (for example, water, ethyl acetate, DMF, acetonitrile and pyridine which may be used alone or in a combination of two or more of these solvents) at room temperature usually for 1 to 6 hours.

When L is hydroxy, Compound (II) is subjected to Mitsunobu reaction.

In Mitsunobu reaction, for example, Compound (II) and 1 to 3 equivalents (preferably 1.1 to 2 equivalents) of Compound (III) are stirred in an inert solvent in the presence of 1 to 2 equivalents of triaryl phosphine (for example, triphenyl phosphine, etc.) and 1 to 2 equivalents of DEAD (diethyl azodicarbonate) usually for 1 to 24 hours.

As the inert solvent, for example, an ether solvent, preferably tetrahydrofuran (THF) is used.

The compound wherein Y is -(CH₂)qCONR⁹(CH₂)r- can be obtained, for example, by reacting carboxylic acid derivative (IV) with amine (V) through an amidation reaction shown in Production Process 2 below. 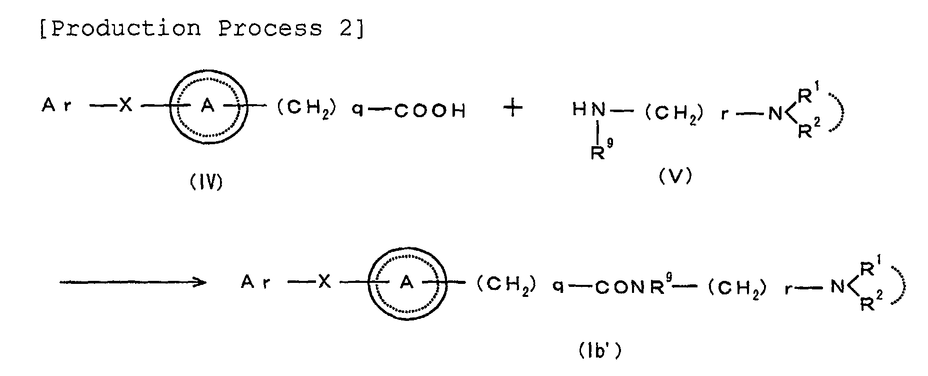 wherein each symbol is as defined above.

### (Step 2)

The "amidation reaction" described above may be carried out in a method known per se, and for example, there can be used (1) a method wherein Compound (IV) is reacted with amine (V) in the presence of a dehydration condensing agent or (2) a method wherein a reactive derivative of Compound (IV) is reacted with amine (V).

In the reaction (1) above, Compound (IV), 1 to 5 equivalents of amine (V) and 1 to 2 equivalents of a dehydration condensing agent are reacted in an inert solvent at room temperature usually for 10 to 24 hours. If necessary, 1 to 1.5 equivalents of 1-hydroxy-1H-benzotriazole (HOBt) and/or 1 to 5 equivalents of a base (for example, triethylamine, etc.) may be added to the reaction.

The "dehydration condensing agent" includes, for example, dicyclohexylcarbodiimide (DCC), 1-ethyl-3-(3-dimethylaminopropyl)carbodiimide hydrochloride (WSC), etc. In particular, WSC is preferable.

The inert solvent includes, for example, a nitrile solvent (preferably acetonitrile), amide solvent (preferably DMF), halogen solvent (preferably dichloromethane), ether solvent (preferably THF), etc., which can be used alone or in a combination thereof.

In the reaction (2) above, a reactive derivative of Compound (IV) and 1 to 5 equivalents (preferably 1 to 3 equivalents) of amine (V) are reacted in an inert solvent, usually at -20 to 50°C (preferably room temperature) for 5 minutes to 40 hours (preferably 1 to 18 hours). The reaction may be carried out if necessary in the presence of 1 to 10 equivalents preferably 1 to 3 equivalents of a base.

The "reactive derivative" of Compound (IV) includes, for example, acid halides (e.g., acid chloride, acid bromide, etc.), mixed acid anhydrides (e.g., acid anhydrides with C₁₋₆ alkyl-carboxylic acid, C₆₋₁₀ aryl-carboxylic acid or C₁₋₆ alkyl carbonic acid, etc.), active esters (e.g., esters with C₁₋₆ alcohols (e.g., methanol, ethanol, 2-propanol, 1-propanol, 1-butanol, etc.), phenol which may be substituted, 1-hydroxybenzotriazole or N-hydroxy succinimide, etc.).

As the "substituent" on the "phenol which may be substituted", there can be used one to five groups such as, for example, a halogen atom, nitro, optionally halogenated C₁₋₆ alkyl and optionally halogenated C₁₋₆ alkoxy. The "phenol which may be substituted" includes, for example, phenol, pentachlorophenol, pentafluorophenol, p-nitrophenol, etc. The reactive derivative is preferably an acid halide or an active ester.

The "base" includes those bases exemplified in step 1 above, and preferable examples are potassium carbonate, sodium carbonate, sodium hydroxide, potassium hydroxide, sodium bicarbonate, sodium bicarbonate, sodium alkoxides (e.g., sodium methoxide, sodium ethoxide, sodium butoxide, etc.), organic amines (e.g., triethylamine, pyridine, triazole, imidazole, hydroxy pyridine, etc.), etc. The inert solvent includes, for example, an alcoholic solvent, an ether solvent, a halogen solvent, an aromatic solvent, a nitrile solvent, an amide solvent, a ketone solvent, a sulfoxide solvent, and water, which can be used alone or in a combination thereof. In particular, methanol, ethanol, acetonitrile, dichloromethane, chloroform, etc. are preferable.

For example, when the active ester is used, the active ester (preferably methyl ester or ethyl ester) is reacted with 1 to 5 equivalents of amine (V) together with a catalytic amount to 2 equivalents of an organic amine (e.g., triethylamine, pyridine, triazole, imidazole, hydroxypyridine, etc.) in an inert solvent.

Preferably, the reaction temperature is room temperature to a temperature under reflux conditions (preferably 50 to 120°C), and the reaction time is 1 to 60 hours. As the inert solvent, an alcohol solvent (e.g., methanol, ethanol, etc.) or the like is used.

Carboxylic acid (IV) and amine (V) used in the reaction are commercially available or easily obtainable. For example, the aromatic acid derivative can be obtained by the production process described in WO 93/24442, etc.

Compound (Ib') thus obtained can be converted into Compound (Ib) by a reaction known per se, for example by a combination of 1 or 2 reactions such as hydrolysis reaction, esterification reaction, amidation reaction, oxidation reaction, reduction reaction and the de-protection reaction described later.

As the "alcoholic solvent", for example, methanol, ethanol, isopropanol, tert-butanol, etc. are used. As the "ether solvent", for example, diethyl ether, tetrahydrofuran (THF), 1,4-dioxane, 1,2-dimethoxyethane, etc. are used. As the "halogen solvent", for example, dichloromethane, chloroform, 1,2-dichloroethane, carbon tetrachloride, etc. are used. As the "aromatic solvent", for example, benzene, toluene, xylene, pyridine, etc. are used. As the "hydrocarbon solvent", for example, hexane, pentane, cyclohexane, etc. are used. As the "amide solvent", for example, N,N-dimethylformamide (DMF), N,N-dimethylacetamide, N-methylpyrrolidone, etc. are used. As the "ketone solvent", for example, acetone, methyl ethyl ketone, etc. are used. As the "sulfoxide solvent", for example, dimethyl sulfoxide (DMSO), etc. are used. As the "nitrile solvent", for example, acetonitrile, propionitrile etc. are used.

When the starting compounds in the above respective reactions have amino, carboxy, hydroxy and carbonyl as substituents, protecting groups used generally in peptide chemistry may have been introduced into each of these groups, and after the reaction, the protecting groups are removed, if necessary, to obtain the objective compound.

The amino-protecting group includes, for example, formyl, C₁₋₆ alkyl-carbonyl (e.g., acetyl, propionyl, etc.), C₁₋₆ alkoxy-carbonyl (e.g., methoxy carbonyl, ethoxy carbonyl, tert-butoxy carbonyl, etc.), benzoyl, C₇₋₁₀ aralkyl-carbonyl (e.g., benzylcarbonyl, etc.), C₇₋₁₄ aralkyloxy-carbonyl (e.g., benzyloxycarbonyl, 9-fluorenylmethoxycarbonyl, etc.), trityl, phthaloyl, N,N-dimethylaminomethylene, silyl (e.g., trimethylsilyl, triethylsilyl, dimethylphenylsilyl, tert-butyldimethylsilyl, tert-butyldiethylsilyl, etc.) and C₂₋₆ alkenyl (e.g., 1-allyl, etc.). Each of these groups may be substituted with one to three substituents such as a halogen atom (e.g., fluorine, chlorine, bromine, iodine, etc.), C₁₋₆ alkoxy (e.g., methoxy, ethoxy, propoxy, etc.), nitro, etc.

The carboxy-protecting group includes, for example, C₁₋₆ alkyl (e.g., methyl, ethyl, propyl, isopropyl, butyl, tert-butyl, etc.), C₇₋₁₁ aralkyl (e.g., benzyl, etc.), phenyl, trityl, silyl (e.g., trimethylsilyl, triethylsilyl, dimethylphenylsilyl, tert-butyldimethylsilyl, tert-butyldiethylsilyl, etc.) and C₂₋₆ alkenyl (e.g., 1-allyl, etc.). Each of these groups may be substituted with one to three substituents such as a halogen atom (e.g., fluorine, chlorine, bromine, iodine, etc.), C₁₋₆ alkoxy (e.g., methoxy, ethoxy, propoxy, etc.), nitro, etc.

The hydroxy-protecting group includes, for example, C₁₋₆ alkyl (e.g., methyl, ethyl, propyl, isopropyl, butyl, tert-butyl, etc.), phenyl, trityl, C₇₋₁₀ aralkyl (e.g., benzyl, etc.), formyl, C₁₋₆ alkyl-carbonyl (e.g., acetyl, propionyl, etc.), benzoyl, C₇₋₁₀ aralkyl-carbonyl (e.g., benzylcarbonyl, etc.), 2-tetrahydropyranyl, 2-tetrahydrofuranyl, silyl (e.g., trimethylsilyl, triethylsilyl, dimethylphenylsilyl, tert-butyldimethylsilyl, tert-butyldiethylsilyl, etc.) and C₂₋₆ alkenyl (e.g., 1-allyl, etc.). Each of these groups may be substituted with one to three substituents such as a halogen atom (e.g., fluorine, chlorine, bromine, iodine, etc.), C₁₋₆ alkyl (e.g., methyl, ethyl, n-propyl, etc.), C₁₋₆ alkoxy (e.g., methoxy, ethoxy, propoxy, etc.), nitro, etc.

The carbonyl-protecting group includes, for example, cyclic acetal (e.g., 1,3-dioxane, etc.) and non-cyclic acetal (e.g., di-C₁₋₆ alkylacetal, etc.).

These protecting groups can be removed according to a method known per se, for example the method described in Protective Groups in Organic Synthesis, John Wiley and Sons (1980). For example, a method of using an acid, a base, UV rays, hydrazine, phenyl hydrazine, sodium N-methyldithiolcarbaminate, tetrabutyl ammonium fluoride, palladium acetate or a trialkylsilyl halide (for example, trimethylsilyl iodide, trimethylsilyl bromide, etc.), reduction, etc., are used.

Compound (Ib) can be isolated and purified by a means known per se, for example solvent extraction, conversion of liquid properties, transfer to other solvent, crystallization, re-crystallization, chromatography and the like. Further, the starting compound of Compound (Ib), or a salt thereof, can be isolated and purified according to the same known means as described above, but may also be subjected as the starting material in the subsequent step as it is without isolation.

When Compound (Ib) contains an optical isomer, stereoisomer, position isomer and rotational isomer thereof, these isomers are also included in Compound (Ib), and each of the isomers can be obtained as an isolated substance by a synthesis method and separation means known per se. For example, when there is an optical isomer in Compound (Ib), a resolved optical isomer is also included in Compound (Ib).

The optical isomer can be produced by a method known per se. Specifically, the optical isomer is obtained by using an optically active synthetic intermediate or by optical resolution of a final racemic mixture by a conventional method.

As the method of optical resolution, a method known per se, for example a fractional re-crystallization method, a chiral column method, a diastereomer method, etc. are used.

### 1) Fractional re-crystallization method

The racemic modification is reacted with an optically active compound (for example, (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, or the like)) to form a salt thereof, which are then separated by fractional re-crystallization method and subjected, if necessary, to a neutralization step, whereby each of the free optical isomers is obtained.

### 2) Chiral column method

A method of separating the racemic modification or a salt thereof by an optical isomer-separating column (chiral column). For example, when liquid chromatography is used, a mixture of optical isomers is added to a chiral column such as ENANTIO-OVM (Tosoh Corporation) or CHIRAL series produced by Daicel Ltd. and developed with water, a buffer (for example, a phosphate buffer) or an organic solvent (for example, ethanol, methanol, isopropanol, acetonitrile, trifluoroacetic acid, diethylamine, etc.) or a mixture thereof, whereby the optical isomers are separated from each other. For example, in case of gas chromatography, separation can be carried out by using a chiral column such as CP-Chirasil-DeX CB (G. L. Science).

### 3) Diastereomer method

A method wherein a racemic mixture is chemically reacted with an optically active reagent to form a mixture of diastereomers, and the diastereomers are then separated from each other by a conventional separation means (for example, fractional re-crystallization, chromatography, etc.) and the optically active reagent site of thus isolated substance is removed by chemical treatment such as hydrolysis reaction, whereby each of the optical isomers is obtained. For example, when Compound (I) has hydroxy or primary or secondary amine in the molecule thereof, the compound is subjected to condensation reaction with an optically active organic acid (for example, MPTA [α-methoxy-α-(trifluoromethyl)phenylacetic acid], (-)-menthoxyacetic acid, etc.) or the like, whereby diastereomers thereof in an ester or amide form can be obtained. On one hand, when Compound (I) has a carboxylic acid group, the compound is subjected to condensation reaction with an optically active amine or an alcohol reagent, whereby diastereomers thereof in an amide or ester form are obtained. The diastereomers are separated from each other, and each diastereomer is subjected to acid hydrolysis or alkali hydrolysis thereby converting it into each optical isomer of the original compound.

Compound (I) (hereinafter referring to Compound (I) including Compounds (I'), (Ia) and (Ib) in addition to Compound (I)) has an excellent inhibitory activity of β secretase, and is thus useful for preventing and treating (1) nerve degenerative diseases (e.g., senile dementia, Alzheimer's disease, Down's syndrome, Parkinson's disease, Creutzfeldt-Jakob disease, amyotrophic spinal lateral sclerosis, diabetic neuropathy, etc.), (2) nerve disorders at the time of cerebrovascular disorders (e.g., cerebral circulation insufficiency accompanying cerebral infarction, cerebral hemorrhage, cerebral arteriosclerosis, etc.), at the time of head or spinal cord injuries, at the time of the sequelae of encephalitis or at the time of cerebral palsy, (3) memory impairment (e.g., senile dementia, amnesia, etc.), or (4) psychiatric disorders (e.g., depression, psychasthenia, schizophrenia, etc.) in which β secretase is involved.

Further, Compound (I) is also useful by inhibiting β secretase to inhibit production and secretion of β amyloid protein and/or by promoting secretion of sAPPα for preventing and treating (1) nerve degenerative diseases, (2) nerve disorders at the time of cerebrovascular disorders, at the time of head or spinal cord injuries, at the time of the sequelae of encephalitis, or at the time of cerebral palsy, (3) memory impairment, or (4) psychiatric disorders.

Further, Compound (I) is also useful as a neurotrophic factor-like agent and useful in preventing and treating (1) nerve degenerative diseases, (2) nerve disorders at the time of cerebrovascular disorders, at the time of head or spinal cord injuries, at the time of the sequelae of encephalitis, or at the time of cerebral palsy, (3) memory impairment, or (4) psychiatric disorders in which a neurotrophic factor is involved.

Further, Compound (I) is also useful in preventing and treating nerve disorders at the time of head or spinal cord injuries, at the time of the sequelae of encephalitis or at the time of cerebral palsy, or psychiatric disorders (for example, depression, psychasthenia, schizophrenia, etc.).

In prevention and treatment of the diseases described above, Compound (I) can also be used in combination with e.g. therapeutic agents for Alzheimer's diseases (for example, choline esterase inhibitors such as donepezil, rivastigmine, galanthamine, TAK-147, etc. and cerebral function activators such as Idebenone, Memantine, vinpocetine, etc.), anti-Parkinson drugs (for example, L-dopa, Deprenyl, carbidopa + levodopa, Pergolide, Ropinirole, cabergoline, Pramipexol, Entacapone, Lazabemide, etc.), therapeutic agents for amyotrophic spinal lateral sclerosis (for example, riluzole, mecasermin, Gabapentin, etc.), neurotrophic factors, anti-depressants (for example, fluoxetine, Sertraline, paroxetine, Venlafaxine, Nefazodone, levoxetine, imipramine hydrochloride, Duloxetine, etc.), therapeutic agents for schizophrenia (for example, Olanzapine, risperidone, Quetiapine, Iloperidone, etc.), etc.

Further, Compound (I) is low toxic and excellent to penetrate into the brain.

Accordingly, Compound (I) is useful as an agent for safely preventing and treating diseases attributable to β secretase in mammals (e.g., rats, mice, guinea pigs, rabbits, sheep, horses, pigs, cattle, monkeys, humans, etc.).

Compound (I) can be formed into a pharmaceutical preparation in a means known per se, and Compound (I) can be safely administered orally or parenterally (for example, through topical, rectal, nasal or intravenous administration) as it is or as a pharmaceutical composition in the form of e.g. tablets (sugar-coated tablets, film-coated tablets, etc.), powders, granules, capsules (including soft capsules), liquid formulations, injections, suppositories, sustained release agents, etc. which are prepared by suitably mixing Compound (I) with a suitable amount of pharmacologically acceptable carriers in the pharmaceutical manufacturing process.

The content of Compound (I) in the pharmaceutical composition of the present invention is usually about 0.1 to 100% by weight based on the whole composition. The dose is varied depending on the subject of administration, administration route, intended diseases, etc., and when used as the agent for treating Alzheimer's disease, the active ingredient (Compound (I)) can be administered orally to a man (about 60 kg) in an amount of about 0.01 to 500 mg, preferably about 0.1 to 100 mg, more preferably 1 to 100 mg in one portion or several divided portions every day.

The pharmaceutically acceptable carriers used in production of the pharmaceutical composition of the present invention include a wide variety of organic or inorganic carrier materials ordinarily used as pharmaceutical materials, and examples thereof include excipients, lubricants, binders or disintegrators in solid preparations, or solvents, solubilizers, suspension agents, isotonizing agents, buffers and analgesics in liquid preparations. If necessary, additives such as preservatives, antioxidants, coloring agents, sweeteners, adsorbents, wetting agent, etc. can also be used.

The excipients used include, for example, lactose, white sugar, D-mannitol, starch, corn starch, microcrystalline cellulose, light silicic anhydride, etc.

The lubricants used include, for example, magnesium stearate, calcium stearate, talc, colloidal silica, etc.

The binders used include, for example, microcrystalline cellulose, white sugar, D-mannitol, dextrin, hydroxypropyl cellulose, hydroxypropylmethyl cellulose, polyvinyl pyrrolidone, starch, sucrose, gelatin, methyl cellulose, sodium carboxymethyl cellulose, etc.

The disintegrators used include, for example, starch, carboxymethyl cellulose, calcium carboxymethyl cellulose, sodium croscarmellose, sodium carboxymethyl starch, L-hydroxypropyl cellulose, etc.

The solvents used include, for example, injection water, alcohol, propylene glycol, Macrogol, sesame oil, corn oil, etc.

The solubilizers used include, for example, polyethylene glycol, propylene glycol, D-mannitol, benzyl benzoate, ethanol, trisaminomethane, cholesterol, triethanolamine, sodium carbonate, sodium citrate, etc.

The suspension agents used include, for example, surfactants such as stearyl triethanolamine, sodium laurylsulfate, lauryl aminopropionic acid, lecithin, benzalconium chloride, benzetonium chloride and glycerine monostearate, and hydrophilic polymers such as polyvinyl alcohol, polyvinyl pyrrolidone, sodium carboxymethyl cellulose, methyl cellulose, hydroxymethyl cellulose, hydroxyethyl cellulose, hydroxypropyl cellulose, etc.

The isotonizing agents used include, for example, glucose, D-sorbitol, sodium chloride, glycerine, D-mannitol, etc.

The buffers used include, for example, buffers such as phosphates, acetates, carbonates, citrates, etc.

The analgesics used include, for example, benzyl alcohol, etc.

The preservatives used include, for example, paraoxybenzoates, chlorobutanol, benzyl alcohol, phenetyl alcohol, dehydroacetic acid, sorbic acid, etc.

The antioxidants used include, for example, sulfites, ascorbic acid, etc.

Hereinafter, the present invention is illustrated in more detail by reference to the Reference Examples, Examples and Test Examples, but these examples are described for illustrative purposes and are not intended to limit the present invention, and may be modified without departure from the scope of the present invention.

In the Reference Examples and Examples below, the "room temperature" refers to 0 to 30 °C, and the organic solvent was dried over magnesium sulfate anhydride or sodium sulfate anhydride. The "%" means % by weight unless otherwise specified.

Infrared absorption spectra were taken with a Fourier transform infrared spectrophotometer by a diffuse reflection method.

The meanings of abbreviations used in the specification are as follows:
s: singlet
d: doublet
t: triplet
q: quartet
m: multiplet
br: broad
dd: double doublet
J: coupling constant
Hz: Hertz
CDCl₃: heavy chloroform
THF: tetrahydrofuran
DMF: N,N-dimethyl formamide
DMSO: dimethyl sulfoxide
¹H-NMR: proton nuclear magnetic resonance (measured in a free form in CDCl₃)
IR: infrared absorption spectrum
DMSO-d₆: heavy dimethyl sulfoxide
WSC: 1-ethyl-3-(3-dimethylaminopropyl)carbodiimide hydrochloride
HOBt: 1-hydroxy-1H-benzotriazole
IPE: diisopropyl ether
DMAP: 4-dimethylaminopyridine
WSCD: 1-ethyl-3-(3-dimethylaminopropyl)carbodiimide

### Reference Example 1

### (+)-N,N-Dimethyl-(6-hydroxy-2-tetralin)acetamide

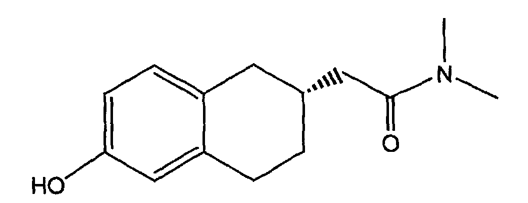

DL-Methionine (362.8 g) and (+)-N,N-dimethyl-(6-methoxy-2-tetralin)acetamide (546.0 g) were added by portions to methanesulfonic acid (1638 mL) and dissolved therein at room temperature. The mixture was reacted with heating at an internal temperature of 110°C for 8 hours under a nitrogen stream. The reaction mixture was cooled to an internal temperature of 10 °C, and methanol (2730 mL), cold water (1092 mL) and 25 % cold ammonia water were added dropwise in turn to adjust the mixture to pH 7.0. After 1 hour at 30°C, the precipitated crystals were collected by filtration and washed twice with a mixture of methanol and tap water (1 : 2) (1640 mL). The product was dried at 50°C under reduced pressure until the weight became constant to obtain the title compound (475.3 g, yield: 87.7 %) as yellow crystals.
¹H-NMR (300MHz, DMSO-d₆) δ : 1.32-1.36(1H, m), 1.82-1.86(1H, m), 2.04-2.08(1H, m), 2.22-2.32(3H, m), 2.63-2.74(3H, m), 2.83(3H, s), 2.96(3H, s), 6.45-6.50(2H, s), 6.79(1H, d, J=8.1Hz), 8.96(1H, s).

### Reference Example 2

### (+)-N,N-Dimethyl-(6-(4-biphenylyl)methoxy-2-tetralin)acetamide

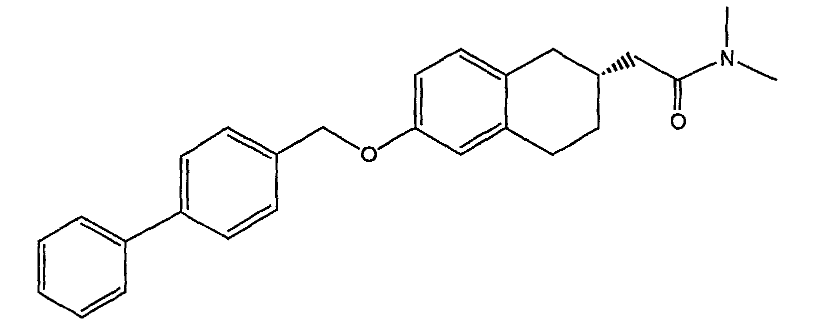

4-Hydroxymethylbiphenyl (378.6 g) was dissolved in DMF (1133 mL), and thionyl chloride (177.6 mL) was added dropwise thereto at an internal temperature of 20°C or lower. The mixture was reacted at room temperature for 1.5 hours. Ethyl acetate (2267 mL) was added to the reaction mixture and cooled to 10°C, and tap water (1133 mL) was added dropwise thereto at 20°C or lower. The organic layer was separated and washed in turn with 10% aqueous sodium carbonate (1133 mL), 5% aqueous sodium bicarbonate (1133 mL) and water (1133 mL). The organic layer was separated and concentrated under reduced pressure such that the amount of the residual mixture became 763 g, then DMF (872 mL) was added thereto, the mixture was concentrated again under reduced pressure, and the residual ethyl acetate was distilled off to obtain a solution of 4-chloromethyl biphenyl in DMF (1286 g) (content: 32.1%; yield: 99.1%). To this DMF solution were added (+)-N,N-dimethyl-(6-hydroxy-2-tetralin)acetamide (435.9 g), potassium carbonate (516.4 g) and DMF (436 mL), and the mixture was stirred at an internal temperature of 80°C for 3 hours under a nitrogen stream. Methanol (1308 mL) was added to the reaction mixture, and water (1744 mL) was added dropwise thereto, while maintaining the internal temperature at about 60°C, followed by stirring at 60°C for 30 minutes. After further stirring at 40°C for 1 hour, the precipitated crystals were collected by filtration and washed twice with methanol (1744 mL) and then water heated at 40°C (2180 mL). The crystals were dried at 50°C under reduced pressure to obtain the title compound (726.8 g, yield: 96.7 %) as pale yellow crystals.
¹H-NMR (300MHz, CDCl₃) δ : 1.42-1.48(1H, m), 1.97-2.04(1H, m), 2.30-2.47(4H, m), 2.79-2.91(3H, m), 2.97(3H, s) , 3.01(3H, s) , 5.06(2H, s), 6.73-6.78(2H, m), 6.97 (1H, d, J=8.3Hz), 7.34-7.62(9H, m).

### Reference Example 3

### (R)-(+)-6-(4-Biphenylyl)methoxy-2-[2-(N,N-dimethylamino)ethyl]tetralin hydrochloride·1H₂O (Compound A)

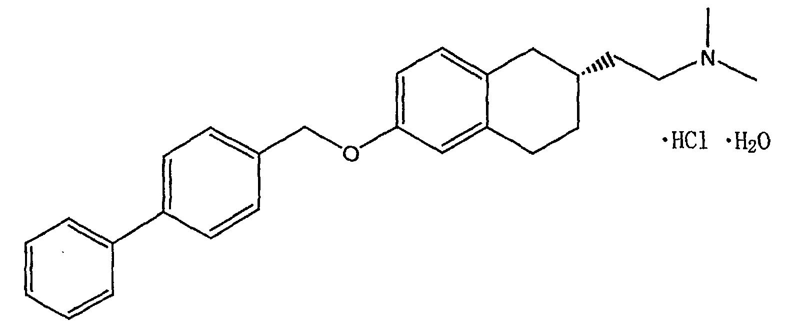

(+)-N,N-Dimethyl-(6-(4-biphenylyl)methoxy-2-tetralin)acetamide (695 g) was suspended in toluene (3475 mL), and sodium dihydro-bis(2-methoxyethoxy)aluminate (70 % solution in toluene) (562 g) was added dropwise thereto at an internal temperature of 20°C or lower under a nitrogen stream. After the mixture was stirred at room temperature for 1.5 hours, 4 N aqueous sodium hydroxide (695 mL) was added thereto at 20°C or lower, and the mixture was stirred at room temperature for 30 minutes and separated into layers. The organic layer was washed twice with 1 N aqueous sodium hydroxide (695 mL) and twice with water (1390 mL). Toluene (348 mL) was added to the organic layer and heated at 60°C, and conc. hydrochloric acid (content: 36 %) (175 mL) was added dropwise thereto. The reaction mixture was stirred with ice-cooling for 1 hour, and the precipitated crystals were collected by filtration and washed with toluene (695 mL) and 50% aqueous methanol (1390 mL). The crystals were dried at 40°C under reduced pressure to obtain the title compound (723 g, yield: 94.4 %) as pale yellow crystals.
¹H-NMR (300MHz, DMSO-d₆) δ : 1.32-1.40( 1H, m), 1.62-1.74(3H, m), 1.82-1.90(1H, m), 2.28-2.38(1H, m), 2.74(6H, s), 2.76-2.82(3H, br), 3.08-3.16(2H, m), 5.09(2H, s), 6.72-6.80(2H, m), 6.96(1H, d, J=8.0Hz), 7.32-7.38(1H, m), 7.44-7.54(4H, m), 7.64-7.72(4H, m), 10.4(1H, br).

### Reference Example 4

### 4-(4-Biphenylylmethoxy)phenylacetic acid

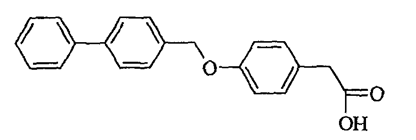

Potassium carbonate (2.5 g) and 4-phenylbenzyl chloride (4 g) were added to a solution of ethyl 4-hydroxyphenylacetate (3.6 g) in DMF (50 ml). The reaction solution was stirred at 60°C for 6 hours and then poured into water. The resultant crystals were suspended in ethyl acetate, washed with water and concentrated. The resultant crude crystals were dissolved in THF (100 ml) and ethanol (50 ml), and 2 N sodium hydroxide (20 ml) was added thereto. The reaction mixture was stirred for 18 hours with heating at 60°C and then concentrated. The residues were acidified with 2 N hydrochloric acid, and the resultant crystals were collected by filtration and washed with ethyl ether to obtain the title compound (5.3 g).
Melting point: 170-171°C

### Reference Example 5

### 4-(4-Biphenylylmethoxy)phenyl-N-[2-(N,N-dimethylamino)ethyl] acetamide (Compound B)

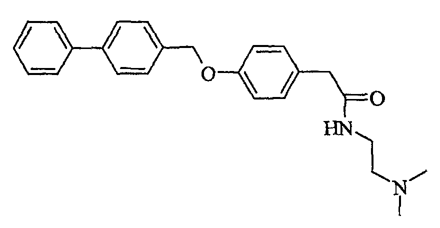

WSC (0.4 g) and HOBt (0.3 g) were added to a solution of 4-(4-biphenylylmethoxy)phenylacetic acid (0.6 g) in THF (30 ml). N,N-dimethylethylene diamine (0.2 g) was added to the reaction mixture. The mixture was stirred at room temperature for 18 hours, and the reaction mixture was poured into water and extracted with ethyl acetate. The organic layer was washed with water, dried and concentrated. The residues were recrystallized from ethyl acetate/ethanol to obtain the title compound (0.3 g).
Melting point: 160-161°C

### Preparation Example 1

| | |
|---|---|
| (1) Compound A | 50 mg |
| (2) Lactose | 34 mg |
| (3) Corn starch | 10.6 mg |
| (4) Corn starch (paste) | 5 mg |
| (5) Magnesium stearate | 0.4 mg |
| (6) Calcium carboxymethyl cellulose | 20 mg |
| total | 120 mg |

According to a conventional method, the above (1) to (6) were mixed and tableted by a tableting machine to give tablets.

### Preparation Example 2

### Film-coated tablets containing Compound A

### Formulation:

**Table 1**

| Composition | Amounts (mg) |
|---|---|
| Compound A | 8.0 |
| D-mannitol | 74.0 |
| Corn starch | 14.3 |
| Hydroxypropyl cellulose | 3.0 |
| Magnesium stearate | 0.7 |
| Total (bare tablets) | 100.0 |
| | |
| Bare tablets | 100.0 |
| (Film components) | |
| Hydroxypropylmethyl cellulose | 3.592 |
| 2910 | |
| Titanium oxide | 0.4 |
| Yellow iron sesquioxide | 0.008 |
| Total | 104.0 |

Compound A (440 g), D-mannitol (4070 g) and corn starch (605 g) were mixed uniformly in a fluidized bed granulation drying machine (FD-5S, Paurek Co., Ltd.), then sprayed in the machine with an aqueous solution prepared by dissolving hydroxypropyl cellulose (HPC-L) (165 g), granulated and dried in the fluidized bed granulation drying machine. The resultant granules were milled with a power mill and screened with a 1.5 mmφ punching screen to give uniform particles. Corn starch (161.7 g) and magnesium stearate (34.3 g) were added to the resultant uniform particles (4704 g) and mixed in a tumbler mixer to give granules for tableting, which were then tableted in a weight of 100 mg per tablet by a 6.5 mmφ millstone, to prepare bare tablets.

Hydroxypropylmethyl cellulose 2910 (TC-5 (trade name), produced by Shin-Etsu Chemical Co., Ltd.) was dissolved in and mixed with an aqueous suspension of titanium oxide and yellow iron sesquioxide. The resulting mixture was sprayed onto the bare tablets in a coating machine (DRC-500), to give about 42000 film-coated tablets each containing 8 mg of Compound A.

### Test Example 1

### Measurement of inhibitory activity on β secretase

The gene manipulation procedures using Escherichia coli were carried out according to the methods described in the Molecular Cloning.

### (1) Construction of expression plasmid of human β secretase

Because there was one-base insertion (102-position) in a nucleotide sequence of clone number FG04087 (GenBank Accession No. AB032975, Kazusa DNA Research Institute) as a nucleotide sequence of a gene coding for β secretase as compared with a nucleotide sequence reported by Bennett et al. (Science 286, 735-741 (1999)), conversion of the nucleotide sequence was carried out, and a nucleotide sequence (5'-GATTACAAGGATGACGACGATAAG-3' (SEQ ID NO:1)) coding for Flag peptide (Asp-Tyr-Lys-Asp-Asp-Asp-Asp-Lys (SEQ ID NO:8)) was added to the C-terminus of the nucleotide sequence to facilitate purification. First, 20 pmol each of a pair of primers [5'-GGCACCACCAACCTTCGT-3' (SEQ ID NO:2) and 5'-GGTACCTACTTATCGTCGTCATCCTTGTAATCCTTCAGCAGGGAGATGTCATCAG-3' (SEQ ID NO:3) containing the nucleotide sequence coding for Flag peptide] prepared on the basis of the nucleotide sequence of β secretase gene reported by Bennett et al. were added to a gene of clone number FG04087 as the template, and the mixture was subjected to PCR reaction using KOD (Toyobo) and MiniCycler™ (MJ Research) (reaction conditions: 1 cycle at 94°C for 2 minutes, 3 cycles at 98°C for 15 seconds, at 72°C for 2 seconds, 74°C for 10 seconds, 3 cycles at 98°C for 15 seconds, at 68°C for 2 seconds, 74°C for 10 seconds, 3 cycles at 98°C for 15 seconds, at 64°C for 2 seconds, 74°C for 10 seconds, and 28 cycles at 98°C for 15 seconds, at 60°C for 2 seconds, 74°C for 10 seconds). The PCR product was subjected to agarose gel electrophoresis, and about a 700-b DNA fragment was recovered. The fragment was cloned with a Zero Blunt TOPO PCR Cloning Kit (Invitrogen). The resultant plasmid was digested with restriction enzymes APaI (Takara Shuzo) and KpnI (Takara Shuzo) and then subjected to agarose gel electrophoresis to recover about a 250-b DNA fragment. The plasmid containing clone number FG04087 was digested with APaI and then subjected to agarose gel electrophoresis, to recover an about 1.2-kb DNA fragment. These DNA fragments were mixed with an animal cell expression plasmid pcDNA3.1 (-) (Funakoshi) digested with API and KpnI, then ligated by use of Ligation High (Toyobo), and used to transform E. coli JM109 competent cells (Takara Shuzo) to give plasmid pBACE1. For subsequent conversion of the one-base insertion, 20 pmol each of a pair of primers (5'-TAATACGACTCACTATAGGG-3' (SEQ ID NO:4) and 5'-GGCGCCCCCCAGACCACTTCTCAG-3' (SEQ ID NO:5)) prepared on the basis of the nucleotide sequence of β secretase gene reported by Bennett et al. were added to a gene of clone number FG04087 as the template, and the mixture was subjected to PCR reaction using KOD (Toyobo) and MiniCycler™ (MJ Research) (reaction conditions: 1 cycle at 94°C for 2 minutes, 3 cycles at 98°C for 15 seconds, at 72°C for 2 seconds, 74°C for 10 seconds, 3 cycles at 98°C for 15 seconds, at 68°C for 2 seconds, 74°C for 5 seconds, 3 cycles at 98°C for 15 seconds, at 64°C for 2 seconds, 74°C for 5 seconds, and 28 cycles at 98°C for 15 seconds, at 60°C for 2 seconds, 74°C for 5 seconds). The PCR product was subjected to agarose gel electrophoresis, and about a 170-b DNA fragment was recovered. The fragment was cloned with a Zero Blunt TOPO PCR Cloning Kit (Invitrogen). The resultant plasmid was digested with restriction enzymes APaI (Takara Shuzo) and BbeI (Takara Shuzo) and then subjected to agarose gel electrophoresis, to recover about a 120-b DNA fragment. pBACE1 was digested with the same enzymes and subjected to agarose gel electrophoresis to recover about a 1.1-kb DNA fragment. Further, pBACE1 was digested with APaI and subjected to agarose gel electrophoresis to recover about a 5.7-kb DNA fragment. These 3 fragments were ligated by use of Ligation High (Toyobo), and used to transform E. coli JM109 competent cells (Takara Shuzo) to give plasmid pBACE2. The resultant cDNA had the nucleotide sequence set froth in SEQ ID NO:6, and its partial sequence at the 1- to 1527-positions coded for the amino acid sequence set forth in SEQ ID NO:7.

### (2) Expression and purification of recombinant human β secretase in COS7 cells

15 µg of Human β secretase expression plasmid pBACE2 and 45 µl of Fugene 6 (Roche Diagnostics) were allowed to stand in 1.5 ml D-MEM medium for 15 minutes at room temperature, and this mixture was added to COS7 cells previously grown in D-MEM medium (Nikken Seibutsu Igaku Kenkyusho) containing 10 % fetal bovine serum (LifeTech Oriental) in a Tissue culture flask 150ml (Becton, Dickinson & Co). The cells were cultured for 2 days and then recovered, and 5 ml of suspending buffer (0.01 M Tris-HCl (pH 8), 0.15 M NaCl, 1 mM EDTA, 0.5 mM PMSF) was added to the cells which were then disrupted by a sonicator (Tommy Seiko UR-200P) (disruption conditions: output 5, 5 seconds). The disrupted solution was centrifuged (500 g, 10 minutes), the supernatant was ultracentrifuged (100,000 g, 45 minutes), and the precipitates were lyzed (4°C, 2.5 hours) with 0.5 ml lyzing buffer (0.01 M Tris-HCl (pH 8), 0.05 M octyl-β-glucoside, 1 mM EDTA, 0.5 mM PMSF) and then ultracentrifuged (100,000 g, 45 minutes). The supernatant was purified with 100 µl anti-Flag antibody (Sigma). As a result, 4 µg of about 70-kDa recombinant human β secretase as the desired product could be obtained.

### (3) Measurement of inhibitory action on β secretase

25 µl of 0.05 M Acetate buffer (pH 4.5), 10 µl of 250 µM Nma-Ser-Glu-Val-Asn-Leu-Asp-Ala-Glu-Lys(Dnp)-Arg-Arg-NH₂ (SEQ ID NO:9), 10 µl of the recombinant secretase (0.005 mg/ml) obtained in (2) above and 5 µl of 12.3 µM Compound A in 10 % DMF (or 5 µl of 10 % DMF as the control) were put respectively to each well on a 96-well plate (black plate, Corning Ltd.) and incubated at 37°C for 22 hours. After incubation, the fluorescence intensity (excitation wavelength 325 nM, measurement wavelength 460 nM) was measured with a fluoroscanascent (Labosystems Ltd.). Compounds C to J obtained according to the production process described in JP 11-80098 A and the process for producing Compound A in Reference Example 3 were also examined for their inhibitory activity. The results (IC₅₀) are shown in Table 2.

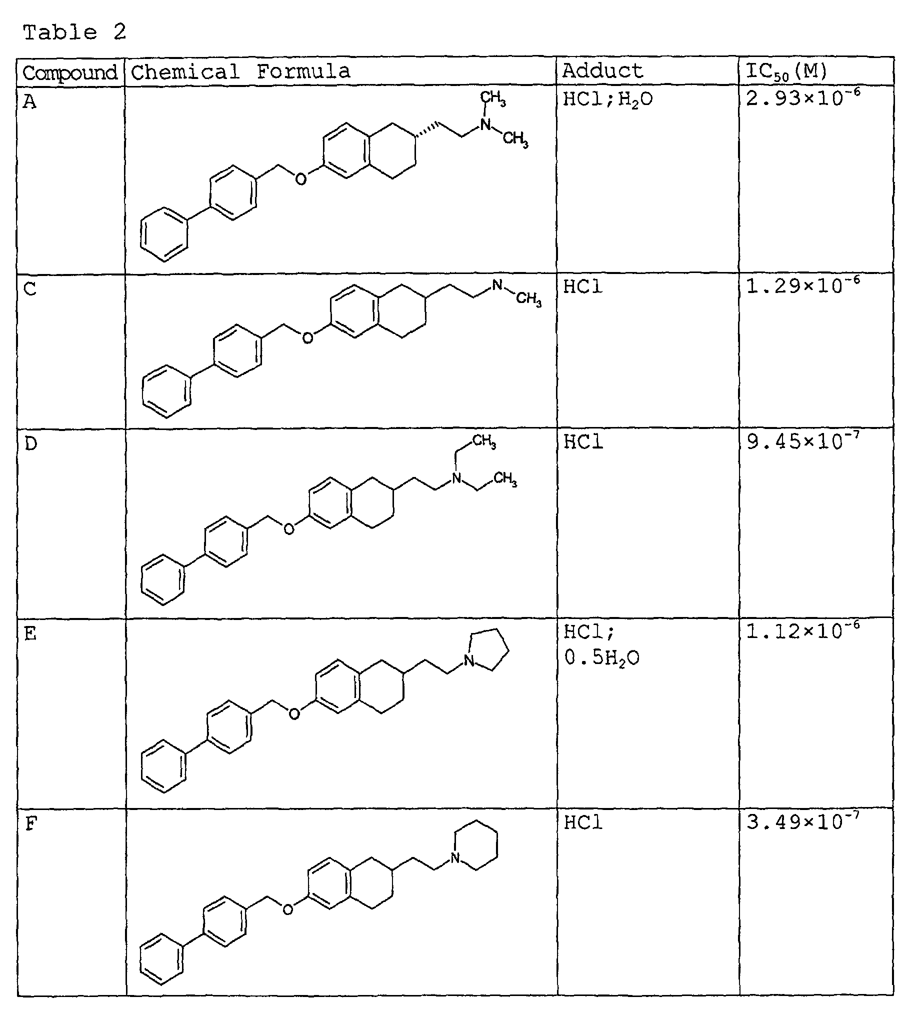

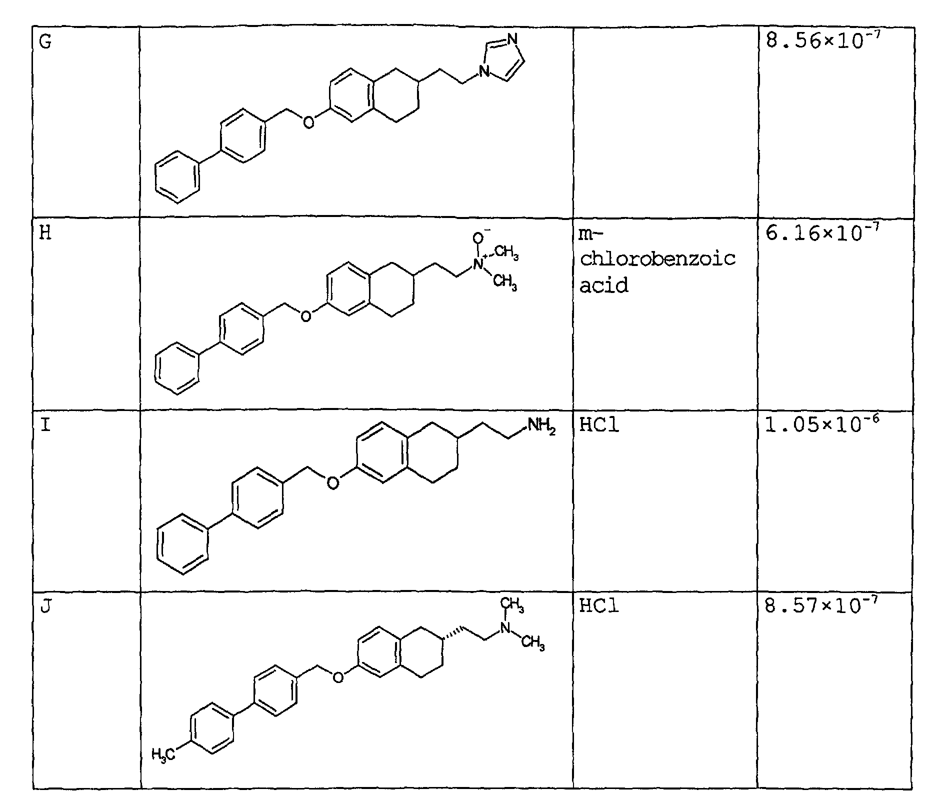

### Industrial Applicability

Compound (I) has an excellent inhibitory activity on β secretase, and is thus useful for preventing and/or treating (1) nerve degenerative diseases (e.g., senile dementia, Alzheimer's disease, Down's syndrome, Parkinson's disease, Creutzfeldt-Jakob disease, amyotrophic spinal lateral sclerosis, diabetic neuropathy etc.), (2) nerve disorders at the time of cerebrovascular disorders (e.g., cerebral circulation insufficiency accompanying cerebral infarction, cerebral hemorrhage, cerebral arteriosclerosis etc.), at the time of head or spinal cord injuries, at the time of the sequelae of encephalitis or at the time of cerebral palsy, (3) memory impairment (e.g., senile dementia, amnesia etc.), or (4) psychiatric disorders (e.g., depression, psychasthenia, schizophrenia etc.), etc., in which β secretase is involved.

### SEQUENCE LISTING FREE TEXT

SEQ ID NO:1
   Designed oligonucleotide encoding FLAG peptide
SEQ ID NO:2
   Primer
SEQ ID NO:3
   Primer comprising DNA sequence encoding Flag peptide
SEQ ID NO:4
   Primer
SEQ ID NO:5
   Primer
SEQ ID NO:8
   FLAG peptide
SEQ ID NO:9
   Designed substance for β secretase

## Claims

1. A β secretase inhibitor comprising a compound represented by the formula: 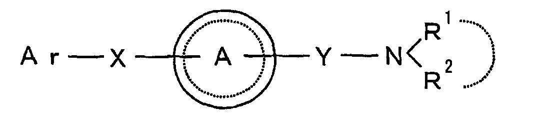 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-_{,} -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or a hydrocarbon group which may be substituted, respectively, or R¹ and R² may, together with the adjacent nitrogen atom, form a nitrogen-containing heterocyclic ring which may be substituted; and ring A represents a ring which may further be substituted, or a salt thereof.

2. The inhibitor according to claim 1 which comprises a compound represented by the formula: 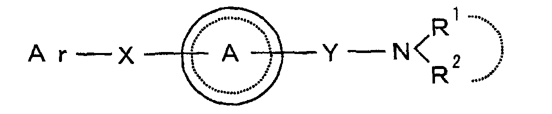 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or C₁₋₆ alkyl which may be substituted, respectively, or R¹ and R² may, together with the adjacent nitrogen atom, form a nitrogen-containing heterocyclic ring which may be substituted; and ring A represents an aromatic ring which may further be substituted, or a salt thereof.

3. The inhibitor according to claim 1 which comprises a compound represented by the formula: 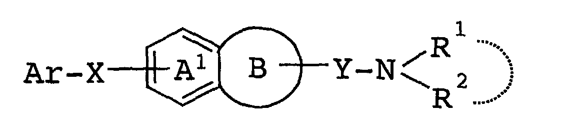 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or C₁₋₆ alkyl which may be substituted, respectively, or R¹ and R² may, together with the adjacent nitrogen atom, form a nitrogen-containing heterocyclic ring which may be substituted; ring A¹ represents benzene ring which may further be substituted, and ring B represents a 4- to 8-membered ring which may further be substituted, or a salt thereof.

4. The inhibitor according to claim 1 which comprises a compound represented by the formula: 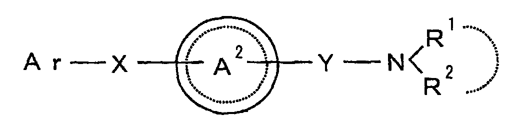 wherein Ar represents an aromatic group which may be substituted; X represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted), a divalent C₁₋₆ aliphatic hydrocarbon group which may contain one or two of these divalent groups, or a bond; Y represents a divalent group selected from -O-, -S-, -CO-, -SO-, -SO₂-, -NR⁸-, -CONR⁸-, -SO₂NR⁸- and -COO- (R⁸ represents hydrogen atom, a hydrocarbon group or acyl which may be substituted) or a divalent C₁₋₆ aliphatic hydrocarbon group which may contain one or two of these divalent groups; R¹ and R² represent hydrogen atom or C₁₋₆ alkyl which may be substituted, respectively, or R¹ and R² may, together with the adjacent nitrogen atom, form a nitrogen-containing heterocyclic ring which may be substituted; and ring A² represents a monocyclic aromatic ring which may further be substituted, or a salt thereof.

5. The inhibitor according to claim 1, wherein the aromatic group represented by Ar is a monocyclic aromatic group, a ring-assembled aromatic group or a condensed aromatic group.

6. The inhibitor according to claim 1, wherein Ar is a ring-assembled aromatic group which may be substituted.

7. The inhibitor according to claim 6, wherein the ring-assembled aromatic group is biphenylyl.

8. The inhibitor according to claim 1, wherein X is -(CH₂)pO- (p is an integer of 1 to 3), -CONH-, -SO₂NH- or C₁₋₃ alkylene.

9. The inhibitor according to claim 1, wherein Y is C₁₋₃ alkylene,-(CH₂)qCONR⁹(CH₂)r- (each of q and r is 0 to 3 and the sum of q and r is an integer of 3 or less, and R⁹ represents hydrogen atom, optionally halogenated C₁₋₆ alkyl or optionally halogenated C₁₋₆ alkyl-carbonyl) or -(CH₂)qCOO(CH₂)r- wherein the symbols are as defined above.

10. The inhibitor according to claim 1, wherein the ring represented by ring A is a monocyclic aromatic ring or a condensed aromatic ring.

11. The inhibitor according to claim 1, wherein the ring A is benzene ring, a 6-membered nitrogen-containing aromatic heterocyclic ring or tetralin ring, each of which may be substituted with a halogen atom and/or C₁₋₆ alkoxy.

12. The inhibitor according to claim 1, wherein the ring A is a benzene ring or tetralin ring, each of which is di-substituted with the group represented by Ar-X- and the group represented by:

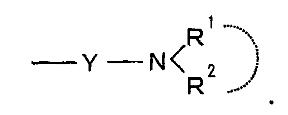

13. The inhibitor according to claim 1, wherein Ar represents biphenylyl; X represents -(CH₂)pO- (p is an integer of 1 to 3), -CONH-, -SO₂NH- or C₁₋₃ alkylene; Y represents C₁₋₃ alkylene, -(CH₂)qCONH(CH₂)r- (each of q and r is 0 to 3 and the sum of q and r is an integer of 3 or less); R¹ and R² represent a hydrogen atom or C₁₋₆ alkyl, respectively, or R¹ and R² may, together with the adjacent nitrogen atom, form a 5- to 6-membered nitrogen-containing heterocyclic ring; and ring A represents benzene ring, a 6-membered nitrogen-containing aromatic heterocyclic ring or tetralin ring, each of which may be substituted with a halogen atom and/or C₁₋₆ alkoxy.

14. The inhibitor according to claim 13, wherein the ring A is benzene ring or tetralin ring, each of which is di-substituted with the group represented by Ar-X- and the group represented by:

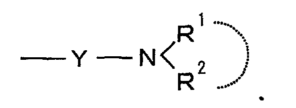

15. The inhibitor according to claim 1 which is an agent for preventing and/or treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment, or (iv) psychiatric disorders, in which β secretase is involved.

16. The inhibitor according to claim 1 which is an agent by promotion of secretion of sAPPα or due to both promotion of secretion of sAPPα and inhibition of production and secretion of β amyloid protein for preventing and/or treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment, or (iv) psychiatric disorders.

17. The inhibitor according to claim 15 or 16, wherein the nerve degenerative disease is Alzheimer's disease or Parkinson's disease.

18. The inhibitor according to claim 1 which is a stimulator of secretion of sAPPα.

19. The inhibitor according to claim 1 which is a neurotrophic factor-like agent.

20. The inhibitor according to claim 1 which is an agent for preventing and/or treating psychiatric disorders or nerve disorders at the time of head or spinal cord injuries, at the time of the sequelae of encephalitis, or at the time of cerebral palsy.

21. Use of the β secretase inhibitor according to claim 1 for the stimulation of an sAPPα secretion promoter, a neurotrophic factor-like agent, or an agent for preventing and/or treating nerve disorders at the time of head or spinal cord injuries, at the time of the sequelae of encephalitis, or at the time of cerebral palsy, or memory impairment or psychiatric disorders.

22. A method for treating (i) nerve degenerative diseases, (ii) nerve disorders at the time of cerebrovascular disorders, at the time of head or spinal cord injuries, at the time of the sequelae of encephalitis, or at the time of cerebral palsy, (iii) memory impairment or (iv) psychiatric disorders in which β secretase is involved, which comprises administrating an effective amount of the β secretase inhibitor according to claim 1 to a mammal.


Sequence Listing
<110> Takeda Chemical Industries, Ltd.
<120> β secretase inhibitor
<130> 662412
<150> JP 2000-152758
<151> 2000-05-19
<210> 1
<211> 24
<212> DNA
<213> Artificial Sequence
<220>
<223> Designed oligonucleotide encoding FLAG peptide
<400> 1
GATTACAAGG ATGACGACGA TAAG 24
<210> 2
<211> 18
<212> DNA
<213> Artificial Sequence
<220>
<223> Primer

<400> 2

GGCACCACCA ACCTTCGT          18

<210> 3

<211> 55

<212> DNA

<213> Artificial Sequence

<220>

<223> Primer comprising DNA sequence encoding Flag peptide

<400> 3

GGTACCTACT TATCGTCGTC ATCCTTGTAA TCCTTCAGCA GGGAGATGTC ATCAG      55

<210> 4

<211> 20

<212> DNA

<213> Artificial Sequence

<220>

<223> Primer

<400> 4

TAATACGACT CACTATAGGG          20

<210> 5

<211> 24

<212> DNA

<213> Artificial Sequence

<220>

<223> Primer

<400> 5

GGCGCCCCCC AGACCACTTC TCAG          24

<210> 6

<211> 1527

<212> DNA

<213> Human

<400> 6

ATGGCCCAAG CCCTGCCCTG GCTCCTGCTG TGGATGGGCG CGGGAGTGCT GCCTGCCCAC     60

GGCACCCAGC ACGGCATCCG GCTGCCCCTG CGCAGCGGCC TGGGGGGCGC CCCCCTGGGG    120

CTGCGGCTGC CCCGGGAGAC CGACGAAGAG CCCGAGGAGC CCGGCCGGAG GGGCAGCTTT    180

GTGGAGATGG TGGACAACCT GAGGGGCAAG TCGGGGCAGG GCTACTACGT GGAGATGACC    240

GTGGGCAGCC CCCCGCAGAC GCTCAACATC CTGGTGGATA CAGGCAGCAG TAACTTTGCA    300

GTGGGTGCTG CCCCCCACCC CTTCCTGCAT CGCTACTACC AGAGGCAGCT GTCCAGCACA    360

TACCGGGACC TCCGGAAGGG TGTGTATGTG CCCTACACCC AGGGCAAGTG GGAAGGGGAG    420

CTGGGCACCG ACCTGGTAAG CATCCCCCAT GGCCCCAACG TCACTGTGCG TGCCAACATT    480

GCTGCCATCA CTGAATCAGA CAAGTTCTTC ATCAACGGCT CCAACTGGGA AGGCATCCTG    540

GGGCTGGCCT ATGCTGAGAT TGCCAGGCCT GACGACTCCC TGGAGCCTTT CTTTGACTCT    600

CTGGTAAAGC AGACCCACGT TCCCAACCTC TTCTCCCTGC AGCTTTGTGG TGCTGGCTTC    660

CCCCTCAACC AGTCTGAAGT GCTGGCCTCT GTCGGAGGGA GCATGATCAT TGGAGGTATC    720

GACCACTCGC TGTACACAGG CAGTCTCTGG TATACACCCA TCCGGCGGGA GTGGTATTAT    780

GAGGTGATCA TTGTGCGGGT GGAGATCAAT GGACAGGATC TGAAAATGGA CTGCAAGGAG    840

TACAACTATG ACAAGAGCAT TGTGGACAGT GGCACCACCA ACCTTCGTTT GCCCAAGAAA    900

GTGTTTGAAG CTGCAGTCAA ATCCATCAAG GCAGCCTCCT CCACGGAGAA GTTCCCTGAT    960

GGTTTCTGGC TAGGAGAGCA GCTGGTGTGC TGGCAAGCAG GCACCACCCC TTGGAACATT
1020
TTCCCAGTCA TCTCACTCTA CCTAATGGGT GAGGTTACCA ACCAGTCCTT CCGCATCACC 1080
ATCCTTCCGC AGCAATACCT GCGGCCAGTG GAAGATGTGG CCACGTCCCA AGACGACTGT 1140
TACAAGTTTG CCATCTCACA GTCATCCACG GGCACTGTTA TGGGAGCTGT TATCATGGAG 1200
GGCTTCTACG TTGTCTTTGA TCGGGCCCGA AAACGAATTG GCTTTGCTGT CAGCGCTTGC 1260
CATGTGCACG ATGAGTTCAG GACGGCAGCG GTGGAAGGCC CTTTTGTCAC CTTGGACATG 1320
GAAGACTGTG GCTACAACAT TCCACAGACA GATGAGTCAA CCCTCATGAC CATAGCCTAT 1380
GTCATGGCTG CCATCTGCGC CCTCTTCATG CTGCCACTCT GCCTCATGGT GTGTCAGTGG 1440
CGCTGCCTCC GCTGCCTGCG CCAGCAGCAT GATGACTTTG CTGATGACAT CTCCCTGCTG 1500
AAGGATTACA AGGATGACGA CGATAAG 1527

<210> 7
<211> 509
<212> PRT
<213> Human

<400> 7
Met Ala Gln Ala Leu Pro Trp Leu Leu Leu Trp Met Gly Ala Gly Val
                5                   10                  15
Leu Pro Ala His Gly Thr Gln His Gly Ile Arg Leu Pro Leu Arg Ser
            20                  25                  30
Gly Leu Gly Gly Ala Pro Leu Gly Leu Arg Leu Pro Arg Glu Thr Asp
        35                  40                  45
Glu Glu Pro Glu Glu Pro Gly Arg Arg Gly Ser Phe Val Glu Met Val
    50                  55                  60
Asp Asn Leu Arg Gly Lys Ser Gly Gln Gly Tyr Tyr Val Glu Met Thr
65                  70                  75                  80
Val Gly Ser Pro Pro Gln Thr Leu Asn Ile Leu Val Asp Thr Gly Ser

                85                  90                  95
Ser Asn Phe Ala Val Gly Ala Ala Pro His Pro Phe Leu His Arg Tyr
            100                 105                 110
Tyr Gln Arg Gln Leu Ser Ser Thr Tyr Arg Asp Leu Arg Lys Gly Val
        115                 120                 125
Tyr Val Pro Tyr Thr Gln Gly Lys Trp Glu Gly Glu Leu Gly Thr Asp
    130                 135                 140
Leu Val Ser Ile Pro His Gly Pro Asn Val Thr Val Arg Ala Asn Ile
145                 150                 155                 160
Ala Ala Ile Thr Glu Ser Asp Lys Phe Phe Ile Asn Gly Ser Asn Trp
                165                 170                 175
Glu Gly Ile Leu Gly Leu Ala Tyr Ala Glu Ile Ala Arg Pro Asp Asp
            180                 185                 190
Ser Leu Glu Pro Phe Phe Asp Ser Leu Val Lys Gln Thr His Val Pro
        195                 200                 205
Asn Leu Phe Ser Leu Gln Leu Cys Gly Ala Gly Phe Pro Leu Asn Gln
    210                 215                 220
Ser Glu Val Leu Ala Ser Val Gly Gly Ser Met Ile Ile Gly Gly Ile
225                 230                 235                 240
Asp His Ser Leu Tyr Thr Gly Ser Leu Trp Tyr Thr Pro Ile Arg Arg
                245                 250                 255
Glu Trp Tyr Tyr Glu Val Ile Ile Val Arg Val Glu Ile Asn Gly Gln
            260                 265                 270
Asp Leu Lys Met Asp Cys Lys Glu Tyr Asn Tyr Asp Lys Ser Ile Val
        275                 280                 285
Asp Ser Gly Thr Thr Asn Leu Arg Leu Pro Lys Lys Val Phe Glu Ala
    290                 295                 300
Ala Val Lys Ser Ile Lys Ala Ala Ser Ser Thr Glu Lys Phe Pro Asp
305                 310                 315                 320

Gly Phe Trp Leu Gly Glu Gln Leu Val Cys Trp Gln Ala Gly Thr Thr
                325                 330                 335
Pro Trp Asn Ile Phe Pro Val Ile Ser Leu Tyr Leu Met Gly Glu Val
            340                 345                 350
Thr Asn Gln Ser Phe Arg Ile Thr Ile Leu Pro Gln Gln Tyr Leu Arg
        355                 360                 365
Pro Val Glu Asp Val Ala Thr Ser Gln Asp Asp Cys Tyr Lys Phe Ala
    370                 375                 380
Ile Ser Gln Ser Ser Thr Gly Thr Val Met Gly Ala Val Ile Met Glu
385                 390                 395                 400
Gly Phe Tyr Val Val Phe Asp Arg Ala Arg Lys Arg Ile Gly Phe Ala
                405                 410                 415
Val Ser Ala Cys His Val His Asp Glu Phe Arg Thr Ala Ala Val Glu
            420                 425                 430
Gly Pro Phe Val Thr Leu Asp Met Glu Asp Cys Gly Tyr Asn Ile Pro
        435                 440                 445
Gln Thr Asp Glu Ser Thr Leu Met Thr Ile Ala Tyr Val Met Ala Ala
    450                 455                 460
Ile Cys Ala Leu Phe Met Leu Pro Leu Cys Leu Met Val Cys Gln Trp
465                 470                 475                 480
Arg Cys Leu Arg Cys Leu Arg Gln Gln His Asp Asp Phe Ala Asp Asp
                485                 490                 495
Ile Ser Leu Leu Lys Asp Tyr Lys Asp Asp Asp Asp Lys
            500                 505

<210> 8
<211> 8
<212> PRT
<213> Artificial Sequence

<220>

<223> FLAG peptide

<400> 8

Asp Tyr Lys Asp Asp Asp Asp Lys          8

<210> 9

<211> 11

<212> PRT

<213> Artificial Sequence

<220>

<223> Designed substrate for β secretase

<400> 9

Ser Glu Val Asn Leu Asp Ala Glu Lys Arg Arg          11